# EUROPEAN PATENT SPECIFICATION

(11) **EP 1 370 679 B1**
(45) Date of publication and mention of the grant of the patent: **05.10.2011**
(21) Application number: 02739090.5
(22) Date of filing: 26.02.2002
(51) Int. Cl.: C12Q 1/00

(54) **METHODS OF MAKING AND USES OF COMPOSITIONS THAT MODULATE INTRONIC REGION-ENCODED PROTEIN FUNCTION**
HERSTELLUNGSVERFAHREN UND VERWENDUNGEN FÜR EINE IM INTRONBEREICH CODIERTE PROTEINFUNKTION MODULIERENDE ZUSAMMENSETZUNGEN
METHODES DE FABRICATION ET UTILISATIONS DE COMPOSITIONS MODULANT LA FONCTION PROTEIQUE CODEE PAR UNE REGION INTRONIQUE

(30) Priority: 01.03.2001 US 272801 P
(43) Date of publication of application: 17.12.2003
(73) Proprietor: Clarity Biosciences, Inc., Encinitas CA 92024 (US)
(72) Inventor: HONEYCUTT, Rhonda, Carlsbad, CA 92009 (US); RALPH, David, Edmond, OK 73034 (US)
(74) Representative: Duxbury, Stephen
(86) International application number: PCT/US2002/006069
(87) International publication number: WO 2002/077160

(56) References cited:
- WO-A1-00/67580
- MEI ET AL.: 'Inhibition of self-splicing group 1 intron RNA: high-troughput screening assays' NUCLEIC ACIDS RESEARCH vol. 24, 15 December 1996, pages 5051 - 5053, XP002956801
- MATSUURA ET AL.: 'A bacterial group II intron encoding reverse transcriptase, maturase and DNA endonuclease activities: biochemical demonstration of maturase activity and insertion of new genetic information within the intron' GENES AND DEVELOPMENT vol. 11, 01 November 1997, pages 2910 - 2924, XP002956802
- ZHANG ET AL.: 'Pentamidine inhibits mitochondrial intron splicing and translation in saccharomyces cerevisiae' RNA vol. 6, 01 July 2000, pages 937 - 951, XP002956803
- DU JARDIN ET AL.: 'Expression of intron-encoded maturase-like polypeptides in potato chloroplasts' CURRENT GENETICS vol. 25, 01 February 1994, pages 158 - 163, XP002956804

## Description

### FIELD OF THE INVENTION

This invention can be used in methods for modulating cellular activity of non-human organisms, and in particular fungi, and methods of identifying and using antifungal agents with improved specificity. More particularly, the technology described herein relates to the identification and use of compounds that target intron-encoded proteins, such as maturases.

### BACKGROUND OF THE INVENTION

Eukaryotic genes consist of alternating series of exons and introns. The exons are sequences that are represented in mRNA, whereas the introns are only present in primary RNA transcripts, also called pre-mRNA, but are removed after transcription to form mature mRNA. Introns are not found in all eukaryotic genes, but are known to exist in the nucleus and organelles of eukaryotic organism. These introns often contain internal open reading frames ("ORFs") that encode proteins that are essential for post-transcriptional RNA processing.

Introns are classified into different groups depending on their structure and function One such function is their ability to encode proteins having homing endonuclease activity, which facilitates the lateral transposition of intronic sequences to other homologous insertion points in a gene. Another function is their ability to encode proteins having maturase activity, which facilitates cleavage of the intronic regions in pre-mRNA. Yet another function is to encode proteins with reverse transcriptase activity. Some introns encode proteins that have one, two or all three of these different activities.

In general, Group I introns encode proteins with endonuclease and/or maturase activity, whereas Group II introns encode proteins with endonuclease, maturase, and/or reverse transcriptase activity. However, most proteins encoded by introns have one or two activities, but not all three. Group I and Group II introns have a wide phylogenetic distribution and appear most often in organellar genomes of organisms. While having related chemistry, the self-recognition of the intron-encoded open reading frames of the RNA sequence that encodes it is highly specific. In addition, many of the intron-encoded proteins belong to a large family with a conserved amino acid motif originally referred to as LAGLIDADG (Hensgens, 1983). These motifs also have four almost identical amino acids, two glycines and two acidic amino acids which are usually aparagines. Together, these features make Group I and Group II introns and the proteins they encode desirable as targets for use in both diagnostic and therapeutic applications.

Microorganisms are the cause of damaging infections in both plants and animals. About 1.3% of patients admitted to hospitals in the U.S. have positive fungal cultures. In particular, *Candida albicans* is one of the most frequently observed pathogens in immunocompromised patients. Most individuals are colonized with *C. albicans* as a commensal organism, and when the individual becomes immunocompromised, the organism can establish an infection. Systemic *Candida* infections extend hospital stays and contribute to increased mortality.

There is a need for epidemiological and diagnostic tools to detect infectious microorganisms in situations where they are hard to distinguish or where the nature of the agent is still under investigation. This is particularly true in fungal diseases where considerable effort has gone into studying and combating such diseases in immunocompromised human patients and in diseases of crops.

Epidemiological and diagnostic tools for classifying plant infecting and mammalian infecting fungi have been used to identify the origin of fungal infections and to track the progression of disease after treatment with antifungal drugs. In the case of mammalian fungal pathogens, there are at least 20 species of *Aspergillus* and at least seven species of *Candida* that cause infection. Almost all the "species" in these genera are defined solely by morphological and nutritional characteristics. These tests are laborious and expensive and have not provided sufficient discrimination to date to classify all infectious organisms.

A variety of detection and identification methods have more recently been developed for detecting *Candida albicans,* including the germ tube test, carbohydrate assimilation test, antigen test, serology, fluorescein-conjugated lectin visualization, and nucleic acid detection by polymerase chain reaction (PCR). Despite these tests, current diagnosis of *Candida* continues to rely on differential culturing, because non-culture tests are costly, requiring multiple enzymatic or hybridization steps and, in the case of PCR, a series of different reaction cocktails and conditions. This additional work diminishes the throughput of a clinical laboratory and increases the chance of error.

There are no less than 30 genera of fungi involved in plant diseases and the relationships among these various species and genera of fungi is still not fully understood. Almost all the "species" in plant fungal genera are presently defined by morphological features or by host range. However, the lack of good morphological characters in fungi has led to often opposing classifications based on host plants, as for in "forma specialis," or other characters for sub-species groupings. Furthermore, in some cases, fungal morphological features can only be discerned when infections are well established on the plant host and symptoms are visible, or when the fungi are present in large enough quantities to be cultured from the plant. Thus, diagnostics of plant infecting fungi is at a rudimentary stage and early detection in asymptomatic plants is not possible using these methods.

Molecular-based methods have been applied to a very limited number of plant pathogenic fungi (reviewed by Swaminathan et al., in Diagnostic Molecular Microbiology, Principles and Applications, DH Persing et al. eds., ASM Press, Washington, DC, pp 26-50 (1993)). For example, immunoassays have been devised for earlier detection of *Pythium* (Miller et al., Phytopathol. 78:1516 (1988)), *Phytophthora* and *Rhizoctonia* (MacDonald et al., Plant Disease 74:655-659 (1990)) and *Mycosphaerella fijiensis* (Novartis, AG Crop Protection Division, Basal Switzerland). Also, commercial kits are available and certified testing laboratories provide enzyme-linked immunoadsorbent assay (ELISA)-based assays for detection of some fungal species.

Furthermore, a variety of nucleic acid protocols have been used to detect plant pathogens, including plasmid content, pulsed field gel electrophoresis, nucleic acid hybridization, restriction digestion, and PCR (reviewed in Maclean et al., Adv. Plant Path., 10:207-244 (1993); van Belkum et al., Clin. Infect. Dis., 18:1017-1019 (1994); and Tang et al., Clin. Chem., 43:2021-2038 (1997)). A few examples of the application of these approaches to fungal pathogens in plants include the arbitrarily primed PCR ("APPCR" or random amplified polymorphic DNA: "RAPD") - based identification for epidemiology and population studies of intersterility groups in *Heterobasidion annosum* (Garbelotto et al., Can. J. Bot., 71:565-569 (1993)) and RAPD-based identification of pathogenic versus non-pathogenic isolates *of Fusarium oxysporum* formal specialis (f. sp.) *dianthi* (Manulis et al., Phytopath., 84:98-101 (1994)).

In addition, probes developed from tandem repeat loci within satellite DNA have been used to detect polymorphisms among *Heterobasidion annosum* isolates (DeScenzo et al., Phytopath., 84:534-540 (1994)).

Although each of these methods are useful, there currently is no single effective approach for detection and classification. Moreover, many of the methods require some foreknowledge of the particular species of organism likely to be present. It is apparent that a need exists for improved molecular methods that avoid the increased costs and reduced speed associated with present diagnostic and epidemiological tests for determining infection of plants and animals.

Related to the need for effective methods for detection and classification of pathogenic organisms, there is also a need to design and implement treatment protocols that are specific for such organisms. Accordingly, the present application provides for new treatment protocols that are based on these methods. Such protocols are based on the discovery that Group I and II introns and the proteins they encode provide an ideal target for the design and use of agents that modulate cellular activity.

Although modulation of eukaryotic RNA splicing reactions has previously been reported as an approach to designing antifungal agents (PCT WO 00/67580), this approach is limited to nuclear-specific splicing reactions that take place in "spliceosomes", which are large macromolecular complexes that catalyze removal of introns. Such complex systems may not provide the most convenient targets for the design of antimicrobial agents. In addition, modulation of eukaryotic RNA autocatalytic splicing reactions by potential antimicrobial agents has also been reported (Nucleic Acids Research 24(24): 5051-5053 (1996).) However, this approach could lead to undesirable cross-reactivities with non-targeted RNAs, including host RNA. In comparison to these two approaches, the present approach does not involve spliceosome mediated reactions whose mechanisms are more complex nor does it involve autocatalytic splicing reactions, which may not provide the desired specificity.

### SUMMARY OF THE INVENTION

The present invention provides for methods as defined in the appended claims that are useful for modulating cellular activity. In one aspect of the invention, a method is provided for screening an agent for modulating cellular activity of a non human organism, wherein said organism contains an intron comprising a nucleic acid encoding a protein that effects IREP (intronic region encoded protein)-mediated post-transcriptional processing ofRNA, said method comprising the steps of: providing the protein in an assay format adapted for studying the effects of the protein on post-transcriptional processing of pre-mRNA; and assaying for said effects in the presence of the agent The intron is preferably organellar, and more preferably a Group I or Group II intron.

The IREP can have any of a mumber of activities associated with IREP, such as restriction endonuclease, reverse transcriptase or maturase activity, but is preferably a maturase.

Introns are found in a variety of organisms, such as fungi, bacteria, plants and protozoa. In one aspect of the present invetion, methods are provided for inhibiting the growth of such organism.

A method is disclosed for screening an agent for modulating IREP-mediated post-transcriptional processing of RNA, said method comprising the steps of: preparing a nucleic acid construct comprising an open reading frame encoding the IREP and a reporter gene functionally associated therewith; expressing protein from the nucleic acid construct; and detecting translation of the reporter gene, wherein a change in translation in the presence of the agent indicates modulation of the IREP-mediated post-transcriptional processing of RNA.

Such screening methods can be carried out in typical assay vessicles in a liquid medium, or can be adapted for use in high-throughput "biochip" formats.

Also disclosed herein are compositions for modulating IREP-mediated post-transcriptional processing of RNA, said composition comprising an agent identified according to the screening methods described above in a pharmaceutically acceptable carrier.

The present invention can be used in a method for modulating cellular activity of a non-human organism associated with a host organism, wherein said non-human organism belongs to a taxonomic group, said method comprising the steps of identifying an IREP specific for the taxonomic group; identifying an agent that modulating IREP-mediated post-tanscriptional processing of RNA; and administering an effective amount of the agent to the host organism. The host organism is a plant.

A pharmaceutical composition is disclosed for inhibiting growth of a non-human organism associated with a host organism, wherein said non-human organism belongs to a taxonomic group of organisms, said compositions comprising: an agent that modulates IREP-mediated post-transcriptional processing ofRNA, wherein said IREP is specific for the taxonomic group; and a pharmaceutically acceptable carrier.

Other objects, features and advantages of the present invention will become apparent from the following detailed description.

### BRIEF DESCRIPTION OF THE DRAWINGS

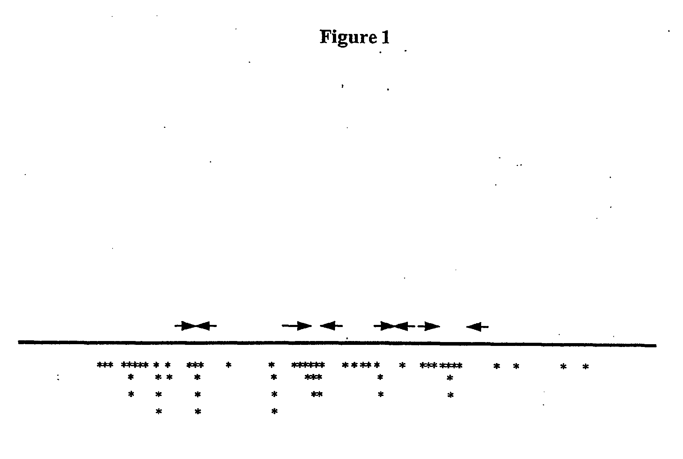

Figure 1 is a schematic representation of the cytochrome oxidase subunit 1 (cox 1) gene showing the location of introns identified by alignment of the *cox* 1 gene from eleven fungal organisms. The solid horizontal line represents the aligned exons (1815 bases drawn to scale), while each asterisk below the line represents an intron insertion. Asterisks aligned in a column represent an intron at the same insertion site in the same gene sequence in multiple organisms. The opposed sets of arrows above the gene identify the locations of intronic region-specific primer pairs.

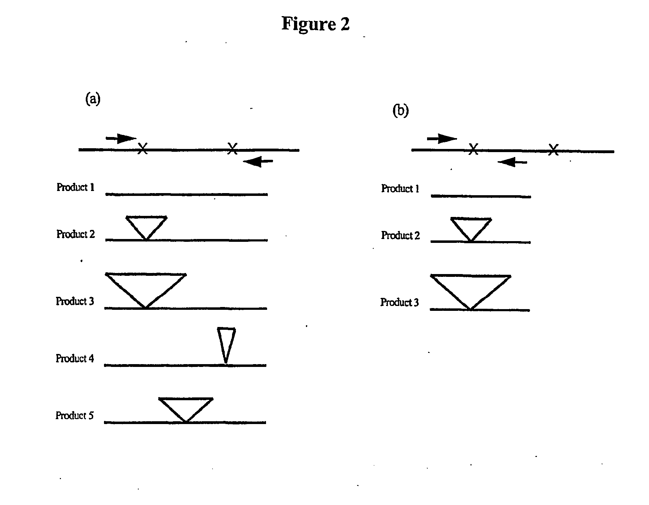

Figure 2A and 2B schematically depict potential PCR products using two examples of intronic region-specific primers in a PCR with template DNA that contains two intron insertion sites (labeled as X and Y). The intronic region-specific primers in Figure 2A are located outside the two intron insertion sites, while in Figure 2B, the primers are located adjacent only one of the two intron insertions sites (i.e., site X).

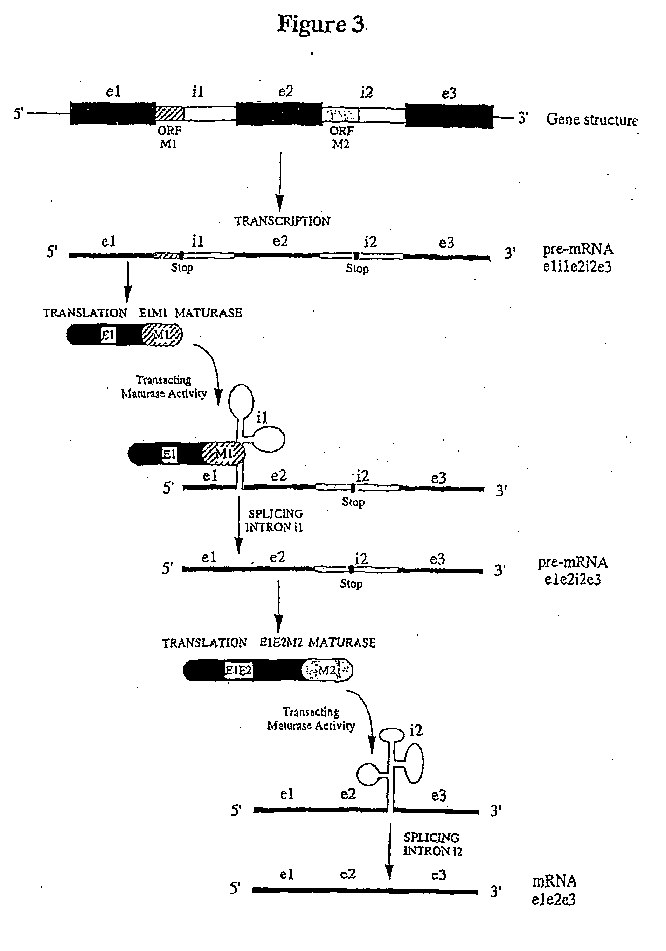

Figure 3 is a schematic representation of intron-encoded maturase-mediated splicing. The gene structure shows 3 exons (e1, e2, and e3) represented by wide dark shaded rectangles interspersed by 2 introns (i1, i2) represented by narrow rectangles. The coding regions portions of maturases (ORFM1, ORFM2) that are located in introns i1 and i2, are represented by striped or gray boxes, respectively. Thick lines represent pre-mRNAs wherein dark and light regions represent the transcribed exons or introns, respectively. The translational stop codons of ORFM1 and ORFM2 are represented by the blacken ovals. The E1M1 and E1E2M2 maturases are represented by oval shapes wherein dark areas correspond to the exon encoded regions (E1 or E1E2) and striped or gray areas correspond to the intron encoded maturases (M1 or M2), respectively. The secondary structures of pre-RNA are indicated by the stem-loop shapes.

### DETAILED DESCRIPTION OF THE INVENTION

The present invention provides novel methods of analyzing nuclear or organellar intronic regions that are useful to distinguish between or among taxonomic groupings of organisms sought to be characterized (i.e., target organisms). As used herein, such methods are collectively referred to as "diagnostic methods". The above methods can be applied to any organism that contains DNA having intronic regions, including fungi, protozoans and other members of the plant and animal kingdoms. Once such intronic regions have been identified, these methods also provide a basis for the design and use of methods to modulate organism-specific cellular activity by affecting intronic region functioning. As used herein, such methods are referred to as "therapeutic methods". For convenience, the remainder of this section, following the subsection entitled "Definitions", is divided into two additional subsections entitled "Diagnostic Methods" and "Therapeutic Methods". It will be apparent that, once appropriate intronic regions and their protein products are identified using diagnostic methods, further characterization of these regions gives rise to new therapeutic methods.

### Definitions

The following definitions are provided to further describe various aspects of the preferred embodiments of the present invention.

Nucleotide: A monomeric unit of DNA or RNA consisting of a sugar moiety (pentose), a phosphate, and a nitrogenous heterocyclic base. The base is linked to the sugar moiety via the glycosidic carbon (1' carbon of the pentose) with the combination of base and sugar referred to as a nucleoside. When the nucleoside contains a phosphate group bonded to the 3' or 5' position of the pentose sugar, it is referred to as a nucleotide. A sequence of linked nucleotides is referred to herein as a "base sequence" or "nucleotide sequence," and their grammatical equivalents, and is represented herein in the conventional left to right orientation being 5'-terminus to 3'-terminus.

Nucleic Acid: A polymer of nucleotides, either single or double stranded.

Polynucleotide: A polymer of single or double stranded nucleotides. As used herein "polynucleotide" and its grammatical equivalents include the full range of nucleic acids. A polynucleotide will typically refer to a nucleic acid molecule comprising a linear strand of two or more deoxyribonucleotides and/or ribonucleotides. The polynucleotides of the present invention include primers, probes, RNA/DNA segments, oligonucleotides or "oligos" (relatively short polynucleotides), genes, vectors, plasmids, and the like.

Gene: A nucleic acid whose nucleotide sequence codes for an RNA or polypeptide. A gene can be either RNA or DNA. A gene also can include intervening segments known as introns.

Complementary Sequence of Nucleotides: A sequence of nucleotides in a single-stranded molecule of DNA or RNA that is sufficiently complementary to a sequence of nucleotides on another single strand of DNA or RNA such that the two strands can hybridize together.

Conserved Sequence of Nucleotides: A nucleotide sequence is conserved with respect to a preselected sequence if the nucleotide sequence can specifically hybridize to an exact complement of the preselected sequence.

Upstream: In the direction opposite to the direction of DNA transcription and, therefore, in a direction from 5' to 3' on the non-coding strand of the DNA, or from 3' to 5' on the mRNA or DNA coding strand.

Downstream: In the direction of DNA transcription and, therefore, in a 3' to 5' direction along the non-coding strand of the DNA or from 5' to 3' on the mRNA or DNA coding strand.

Hybridization: The pairing of substantially complementary nucleotide sequences (strands of nucleic acid) to form a duplex or heteroduplex through formation of hydrogen bonds between complementary base pairs. It is a specific, i.e., non-random, interaction between two complementary polynucleotides.

Hybridization Stringency: Refers to the conditions under which hybridization between two nucleic acid strands is conducted.

High stringency refers to conditions that permit hybridization of only those nucleic acid sequences that form stable hybrids in 0.018M NaCl at 65°C. High stringency conditions can be provided, for example, by hybridization in 50% formamide, 5X Denhardt's solution, 5X sodium chloride- sodium phosphate-Ethylenediaminetetraacetic acid buffer (SSPE buffer), 0.2% sodium dodecyl sulfate (SDS) at 42°C., followed by washing in 0.1X SSPE, and 0.1% SDS at 65°C.

Moderate stringency refers to conditions equivalent to hybridization in 50% formamide, 5X Denhardt's solution, 5X SSPE, 0.2% SDS at 42°C., followed by washing in 0.2X SSPE, 0.2% SDS, at 65°C.

Low stringency refers to conditions equivalent to hybridization in 10% formamide, 5X Denhardt's solution, 6X SSPE, 0.2% SDS, followed by washing in 1X SSPE, 0.2% SDS, at 50°C.

Recipes for Denhardt's solution and SSPE are well known to those of skill in the art as are other suitable hybridization buffers (e.g., Sambrook et al., *supra,* (1989)). For example, SSPE is pH 7.4 phosphate-buffered 0.18M NaCl. SSPE can be prepared, for example, as a 20X stock solution by dissolving 175.3 g of NaCl, 27.6 g of NaH₂PO₄ and 7.4 g ethylenediaminetetraacetic acid (EDTA) in 800 ml of water, adjusting the pH to 7.4, and then adding water to 1 liter. Denhardt's solution (Denhardt, Biochem. Biophys. Res. Commun., 23:641 (1966)) can be prepared, for example, as a 50X stock solution by mixing 5 g Ficoll (Type 400, Pharmacia LKB Biotechnology, Inc., Piscataway, NJ), 5 g polyvinylpyrrolidone, and 5 g bovine serum albumin (Fraction V; Sigma Chem. Co., St. Louis, MO) with 500 ml water and filtering to remove particulate matter.

In the case of PCR, high stringency refers to primer annealing temperatures that are from 0 to 5°C less than the primer Tm. Moderate stringency refers to primer annealing temperatures that are from 5.1 to 10.0°C less than the primer Tm. Low stringency refers to primer annealing temperatures that exceed 10.1°C less than the primer Tm (e.g., 15°C).

Intron: A DNA region that is transcribed into a corresponding region in pre-RNA that is removed during splicing together of protein coding regions ("exons") to form mature messenger RNA.

Intronic Region: DNA sequence comprising an entire intron and some or all of its adjoining upstream and downstream exons, or a portion of an intron with or without some or all of its adjoining upstream exon or some or all of its adjoining downstream exon. The intronic region can be present in nuclear DNA of eukaryotes as well as in organellar DNA from such organelles as mitochondria and chloroplasts and the like. Thus, mitochondrial intronic regions and chloroplastic intronic regions are examples of organellar intronic regions included within the meaning of intronic regions as used herein. Bacterial chromosomal DNA also can contain intronic regions.

Maturase: A protein that facilitates post-transcriptional processing of RNA, which is encoded at least in part by an intronic region.

Amplified Product: Copies of a portion of a DNA sequence and its complementary sequence, which copies correspond in nucleotide sequence to the original DNA sequence and its complementary sequence.

Complement: A DNA sequence that is complementary to a specified DNA sequence.

Primer Site: The segment of the target DNA to which a primer hybridizes.

Primer Extension Reaction: Any of a number of methods that result in the synthesis of a nucleotide sequence from a partially double stranded segment of nucleic acid. A variety of enzymes are known that can add nucleotides to the 3' end of the single stranded segment of the partially double stranded template.

Primer: A polynucleotide, whether purified from a nucleic acid restriction digest or produced synthetically, which is capable of acting as a point of initiation of nucleic acid synthesis when placed under conditions in which synthesis of a primer extension product complementary to a nucleic acid strand is induced, i.e., in the presence of nucleotides and an agent for polymerization such as DNA polymerase, reverse transcriptase and the like, and at a suitable temperature and pH.

Pair of Primers: A 5' upstream primer that hybridizes at the 5' end of the DNA sequence to be amplified and a 3' downstream primer that hybridizes at the 3' end of the sequence to be amplified.

Intronic Region-Specific Primers: A primer pair that amplifies at least one intronic region. The intronic region-specific primer sites can be located in the intron, adjacent upstream and/or downstream exon sequences, upstream or downstream non-adjacent exons or upstream or downstream introns (e.g., Figure 2a) and any combinations thereof.

Homologous Intron: An intron that is present at the same insertion site in the same gene from different organisms without regard to the sequence of the intron.

Primer-Defined Length Polymorphisms (PDLP): Differences in the lengths of amplified DNA sequences due to insertions or deletions in an intronic region that is amplified.

Endonuclease or Restriction Endonuclease: An enzyme that cuts double-stranded DNA of a particular nucleotide sequence called a restriction site. The specificities of numerous endonucleases are well known and can be found in a variety of publications, e.g., Sambrook et al., *supra,* (1989). Endonucleases that produce blunt end DNA fragments by hydrolyzing a phosphodiester bond on both DNA strands at the same site as well as endonucleases that produce sticky ended fragments by hydrolyzing a phosphodiester bond on each strand of the DNA but at separate sites can be used for analysis of DNA sequence differences and for cloning DNA fragments.

Restriction Fragment Length Polymorphism (RFLP): A characterization of DNA nucleotide sequence based on the length of fragments generated when cleaved by a restriction endonuclease.

Primer-Defined Sequence Polymorphisms (PDSP): Differences in the sequences of amplified DNA in an intronic region of the amplified DNA sequence.

Taxon-Specific Intronic Polymorphisms: Differences between and among classical taxonomic groups of organisms. These are based on the polymorphisms defined by the presence, absence of an intron as well as by PDLP and PDSP. As used herein, taxa includes classical groupings such as genus and species, as well as nonclassical groupings which include, for example, species complex, race, subspecies, formal specialis, pathovar, biovar, cultivar and the like.

Target Organisms: Organisms sought to be characterized and whose nucleic acid is used in amplification reactions with intronic region-specific primers to determine polymorphisms based on presence, absence, length or sequence of the intronic region.

Antibody: Any of a large number of proteins of high molecular weight that are produced normally by specialized B type lymphocytes after stimulation by an antigen and act specifically against the antigen in an immune response. Antibodies typically consist of four subunits including two heavy chains and two light chains-also called immunoglobulins. As used herein, antibody includes naturally occurring antibodies as well as non-naturally occurring antibodies such as domain-deleted antibodies, single chain Fv antibodies and the like.

Immunological Binding Reagent: Any type of molecule that is useful to detect a first antibody molecule that binds to a target antigen. An immunological binding reagent can include a labeled second antibody specific for the first antibody or may include avidin or streptavidin when the first antibody is conjugated to biotin. An immunological binding reagent also can be a chemical that has binding specificity for an antibody or other protein.

### Diagnostic Methods

The methods described herein involve selecting an intronic region from a nucleotide sequence of one or more gene homologs. Such intronic regions can be selected by means well known in the art. The intronic regions are then analyzed in DNA of known organisms by a variety of nucleic acid detection methods such as primer extension reactions, separation of amplified products by molecular weight, nucleotide sequencing, or restriction fragment length polymorphism.

In primer extension, intronic region-specific primers suitable for amplifying intronic regions are synthesized and used to amplify the intronic regions in the target organism DNA, if present. The usefulness of a particular intronic region for differentiating between or among taxonomic groupings of target organisms is determined by analyzing the amplified products. Analysis is accomplished, for example, by detecting the presence or absence of the intronic region. Analysis also can be performed by detecting differences in length of the intronic region in the nucleic acid from different organisms (i.e., primer defined length polymorphism; PDLP) or differences in the sequence of the intronic region in the nucleic acid from different organisms (i.e., primer defined sequence polymorphism; PDSP). By analyzing a panel of intronic regions, a taxon-specific, profile of intronic region differences or polymorphisms is identified that can differentiate between or among related species of organisms. Such polymorphisms are useful, for example, to identify all members of a genus or to identify different species of a single genus.

### A. Selecting Intronic Regions Useful for Identifying Organisms

Intronic regions can be selected from sequences obtained from publicly available gene databases such as GOBASE (University of Montreal, Montreal, Canada; http://megasun.bch.umontreal.ca/gobase/), GenBank (National Center for Biotechnology Information, Washington, DC; http://ncbi.nlm.nih.gov/), EMBL (EMBL Outstation-European Bioinformatics Institute, Cambridge, UK, http://www.ebi.ac.uk/embl) or DDBJ (National Institute of Genetics, Mishima, Japan, http:// www.ddbj.nig.ac.jp).

The sequences should be obtained from organisms that are at least broadly taxonomically related to the target organisms sought to be characterized. Such sequences are preferably from organisms within the same kingdom. The gene sequence of the host genome, be it plant, human, or other animal, should be included for comparison, particularly when the sample to be analyzed includes nucleic acid from both the target organism and the host organism (e.g., a blood sample suspected to be infected). For example; if the target organism is a yeast, the gene sequences used to select intronic regions are preferably from fungi.

In fungi, the most conserved mitochondrial genes are the cytochrome oxidase subunit 1 (*cox*1 the apocytochrome b (*cob*)*,* and the ribosomal genes. Sequences of these and other mitochondrial genes are available in GOBASE, which includes, for example, the sequences of mitochondrial genes, *cob*1*, cox*1*, cox*2*, cox*3*, nad*1*, nad*2*, nad*3*, nad*4*, nad*5*, atp6,* and *atp*9*.* These sequences are from subclasses of fungi that have been most extensively studied. Mitochondrial introns have been identified in *cob, cox*1*, cox*2*, nad*1*, nad5,* and other genes.

In addition to public databases, genes with intronic regions also can be cloned and their nucleotide sequence determined (Example 8). Methods for cloning and sequencing genes are well known, including the Sanger dideoxy mediated chain-termination approach and the Maxam-Gilbert chemical degradation approach. These and other nucleic acid sequencing methods are described, for example, in Sambrook et al., Molecular Cloning: A Laboratory Manual, Cold Spring Harbor Laboratory (1989) (chapter 13). Nucleic acid sequencing can be automated using a number of commercially available instruments.

An intronic region can be selected for its ability to differentiate between and among various taxonomic groupings of organisms by a variety of means. An intronic region can be identified, for example, by locating the nucleotide sequence that is present between intronic splice sites in a gene, or aligning the exon(s) of a gene from the nucleotide sequences of at least two organisms that encode the specified gene. Intronic regions also can be identified by comparing cDNA sequence to genomic sequence and by statistical methods to identify sequence motifs and codon usage characteristic of introns. These methods are well known in the art.

When aligning sequences to identify an intronic region, it is important to select gene sequences that contain at least one exon and at least one intron. Sequences without an intron can be used to define a consensus sequence for intronic region-specific primers, but a minimum of two sequences, of which at least one contains an intron, is necessary to identify an intronic region for analysis. The selected gene sequences are aligned according to the exon sequence. Alignment can be accomplished manually or more preferably with a publicly available computer sequence alignment program such as MAP (multiple alignment program) accessible at Baylor College of Medicine (BCM, Houston, TX)) Search Launcher website (http://www.hgsc.bcm.tmc.edu/SearchLauncher/; Smith et al., Genome Res., 6:454-462 (1996)). Alignments can be made from GOBASE by separate downloading of exons and introns, while GenBank accession is usually available as a single genomic sequence.

Once the exons are aligned, the identity and insertion site of the intron can be determined by visual inspection and an intronic region selected. For example, all the exons of a specified gene (e.g., *cox*1) *for* a given organism can be downloaded (e.g., from GOBASE), and fused (in order) into a single file. This process is repeated for each additional organism to be compared. The sequences are then aligned using MAP and the resulting alignments of exons are compared to the genomic sequence to locate intronic insertion sites. In some cases, the intronic sequence is available for confirmation or the exon:intron boundaries are annotated in the database (e.g., GenBank). Primers are then derived to enable detection of intronic polymorphisms.

In some situations, analysis of a single intronic region in the nucleic acid of a target organism will be sufficient to differentiate the organism between or among a particular taxonomic grouping of organisms. More typically, discrimination will require that multiple intronic regions be identified and analyzed. Multiple intronic regions can be identified, for example, by aligning homologous sequences in one or more gene homologs. Multiple intronic regions can be detected using a single primer pair that flanks more than one intron. A homologous intron is one that is present at the same insertion site in the same gene from different organisms without regard to the sequence of the intron). Homologous introns can have the same nucleotide sequence or can have different nucleotide sequences. Such introns are particularly useful for identifying organisms at the subspecies level.

A total of 38 unique intron insertions sites are present in approximately 1400 of the 1800 bases in the consensus alignment of exons from all *cox*1 genes currently known in fungi. Thus, the *cox*1 gene provides a variety of mitochondrial intronic regions to select from a single alignment of sequences (Example 1).

### B. Intronic region-specific Primer Design and Preparation

Intronic regions selected as described herein are evaluated for their use in differentiating between or among selected taxonomic grouping of organisms by, for example, primer extension reactions using intronic region-specific primers. As used herein, intronic region-specific primers refer to a primer pair that is useful for amplifying at least a portion of one intron (i.e., an intronic region). Each primer is complementary to a primer site located in the intron, adjacent upstream and/or downstream exon sequences, upstream or downstream non-adjacent exons or upstream or downstream introns (e.g. Figure 2a) and any combinations thereof. The primer sites are preferably located in conserved sequences.

The intronic region-specific primer sites are generally located upstream and downstream of the intronic region with the 3' end of each primer situated toward the intron insertion site. In this way, the DNA polymerase in the primer extension reaction will generate a copy of the intronic region if it is present in the DNA template.

A primer is preferably single stranded for maximum efficiency, but may alternatively be in double stranded form. If double stranded, the primer is first treated to separate it from its complementary strand before being used to prepare extension products. Preferably, the primer is a polydeoxyribonucleotide. The primer must be sufficiently long to prime the synthesis of extension products in the presence of the agents for polymerization. The exact lengths of the primers will depend on many factors, including temperature and the source of primer.

The primers described herein are selected to be "substantially" complementary to the different strands of each specific sequence to be synthesized or amplified. This means that the primer must be sufficiently complementary to hybridize relatively specifically with its intended primer site in the target template strand. Therefore, the primer sequence may or may not reflect the exact sequence of the template. For example, a non-complementary nucleotide fragment can be attached to the 5' end of the primer, with the remainder of the primer sequence being substantially complementary to the strand. Such non-complementary fragments typically contain an endonuclease restriction site. Alternatively, non-complementary bases or longer sequences can be interspersed into the primer, provided the primer sequence has sufficient complementarity overall with the sequence of the strand to be synthesized or amplified to non-randomly hybridize therewith and thereby form an extension product under polynucleotide synthesizing conditions.

An intronic region-specific primer preferably includes at least about 15 nucleotides, more preferably at least about 20 nucleotides. The primer preferably does not exceed about 30 nucleotides, more preferably about 25 nucleotides, although it can contain fewer nucleotides. Short primer molecules generally require lower temperatures to form sufficiently stable hybrid complexes with the template. Most preferably, the primer includes between about 20 to about 25 nucleotides. The length of the primer will vary inversely with the extent of conservation of the complementary exon sequence. The GC content of the primers should be about 50%.

Intronic region-specific primers are preferably complementary to a primer site located in a conserved region of the gene. Intronic region-specific primers that are based on aligned gene sequences are preferably complementary to a primer site that reflects a consensus of the aligned sequences. The priming or hybridizing region of intronic region-specific primers typically includes the 3'-most (3'-terminal) 15 to 30 nucleotide bases. The 3'-terminal priming portion of each primer is capable of acting as a primer to catalyze nucleic acid synthesis, i.e., initiate a primer extension reaction from its 3' terminus. One or both of the primers can additionally contain a 5'-terminal (5'-most) non-priming portion, i.e., a region that does not participate in hybridization to the preferred template.

The 3'-most base of the primer should be situated either in the first or second position within the codon reading frame so that the 3'-most base is not in a wobble position of a codon. The 3' codon also should be chosen so that there are no redundant bases in the 3'-most position of the primers among coding sequences typical of the kingdom or other taxonomic grouping from which the sequences are derived. Any nucleotides that are not identical to the sequence or its complement are preferably not located at the 3' end of the primer. The 3' end of the primer preferably has at least two, preferably three or more, nucleotides that are complementary to primer site in the template DNA.

In situations where a gene sequence alignment provides multiple potential intronic regions, as in the fungal *cox*1 mitochondrial gene, one may select only a few of the intronic regions for the ability to differentiate between or among the taxonomic groups of interest. Those intronic regions that arise more frequently in the aligned sequences and that exhibit length and/or sequence differences among the aligned sequences are preferred.

One consideration when selecting the location of primer sites is the size of the product produced by primer extension. For example, in one embodiment, the amplifying primer sites are in the exon sequence immediately adjacent to the intron insertion site of the gene. In this case, primer extension will result in a very small sized product (about the combined length of the two primers or so) if the template DNA lacks the intronic region and potentially a much larger product if the template DNA contains the intronic region.

In another approach, the amplifying primers can be located sufficiently far away from the intron insertion site, for example in a non-flanking exon. In this case, primer extension will generate a larger product than in the case when the primer sites directly flank the intronic region. The intronic region-specific primer sites also can be located sufficiently far apart such that they span more than one intron insertion site. In this way, amplification by primer extension can generate a product that contains multiple intronic regions. Although this may complicate the analysis of each intronic region somewhat, this approach has the potential to detect intronic region insertions that were not predicted based on known gene sequence results (e.g., Figure 2A).

Thus, the choice of primer site can affect the size of the product(s) that are produced in a primer extension reaction. Depending on the choice of nucleic acid analysis one can select intronic region-specific primer sites that will produce a particular sized product suited for the analysis method chosen.

Primers can be prepared using a number of methods, including phosphotriester and phosphodiester methods or automated embodiments thereof. The phosphodiester and phosphotriester methods are described in Cruthers, Science, 230:281-285 (1985); Brown et al., Meth. Enzymol., 68:109 (1979); and Nrang et al., Meth. Enzymol., 68:90 (1979). In one automated method, diethylphosphoramidites which can be synthesized as described by Beaucage et al., Tetrahedron letters, 22:1859-1962 (1981) are used as starting materials. A method for synthesizing primer oligonucleotide sequences on a modified solid support is described in U.S. Pat. No. 4,458,066.

### C. Target Organisms and Isolation of Nucleic Acid

Primer extension reactions are preferably performed using purified DNA from the target organism. Isolation of DNA from cells is routine in the art and there are numerous sources of nucleic acid isolation protocols suited for microorganisms such as bacteria and fungi including mammalian cells (e.g., Sambrook et al., *supra,* (1989)). Primer extension reactions also can be performed using DNA that has not been purified but is accessible to the primer. The DNA can be accessible naturally in the sample or can be made accessible following one or more processing steps.

Isolation of fungal DNA can be accomplished by grinding spores in the presence of diatomaceous earth using a Savant grinding instrument (BIO101, San Diego, CA) followed by RNAse treatment, phenol:chloroform extraction, and ethanol precipitation (Zambino et al., Proc. Finnish Forest Res. Instit., 712:297-298 (1998)). Although this method is somewhat time-consuming, the yield and purity are sufficient in PCR with multiple sets of primers.

Other methods for fungal DNA extraction include, Reddy et al., Mol. Cell Probes, 7:121-126 (1993); Bretagne et al., J. Clin. Microbiol., 33:1164-1168 (1995); Verweij et al., J. Clin. Pathol., 48:474-476 (1995); Makimura et al., Med. Microbiol., 40:358-364 (1994); Ausubel et al. in: Current Protocols in Molecular Biology, John Wiley & Sons, NY, pp. 13.11.1-13.11.4 (1994)). Commercial kits such as QIAAMP^{®} (QIAGEN, Inc., Chatsworth, CA: Loffler et al., QIAGEN News, 4:16-17 (1996) and EASY-DNA^{®} (Invitrogen, Inc., Carlsbad, CA) also are available.

Target organisms suitable for identification of intronic regions and for detection by the method disclosed herein include, for example, members of the *Eucaryota* (including *Euglenozoa: trypanosoma)* and Eucaryote Crown Group, subclasses of Fungi/Metazoa Group *(Ascomycota, Basidiomycota, Oomycota, Chytidiomycota,* and *Zygomycota), Avelolata* (e.g. *Toxoplasma), Viridiplantae* (e.g. achloric algae) and various other taxonomic grouping described in the NCBI Taxonomy database (http://www.ncbi.nhn.nih.gov/Taxonomy/tax.html).

Important fungal genera include, for example, *Aspergillus, Candida, Coccidiodes, Cryptococcus, Histoplasma, Blastomyces, Cladosporium Fusarium, Tilletia, Puccinia, Septoria, Botrytis, Pyrenophora,* and *Gaeumannomyces.*

### D. Identifying Intronic Regions

### Types of Intronic Regions

Introns can be classified as either Group I and Group II according to genomic intronic classification (reviewed in Cech, Annu. Rev. Biochem., 59:543-568 (1990); and Perlman et al., Intervening Sequences in Evolution and Development, E. M. Stone and R.J. Schwartz, eds., Oxford Univ. Press, New York (1990)). The groups are distinguished by nucleotide sequence motifs and conserved secondary structure. A fungal species may contain both Group I and Group II introns and the number of introns varies widely between species.

Group I introns are more common in fungal mitochondria, range in length between 200 and 3000 bases, and may contain zero, one, or two open reading frames (ORFs) (Cech, *supra,* (1990)). Some of these ORFs encode proteins of known function including endonucleases and maturases, each having conserved amino acid motifs. Group I ORFs are also mobile elements (Sellem et al., Mol. Evol. Biol., 14:518-526 (1997)).

Group II introns, which are found in fungal mitochondria and more commonly in plant chloroplasts range in length from 900 to 2500 bases. Such introns may contain ORFs encoding for reverse transcriptases (Michel, et al., Annu. Rev. Biochem., 64:435-461 (1995)).

Optional introns are those which are present or absent in the same gene from different species of an organism. Fungi as opposed to insects and other animals have size differences in the mitochondrial genomes which are due in part to the presence of optional introns, and to a lesser extent by intergenic sequences and variation in coding capacity (Belcour et al., Curr. Genet., 31:308-317 (1997)). Introns inserted at identical positions in homologous genes in unrelated species are considered homologous introns even though the intron sequences vary widely.

The insertion positions of some mitochondrial introns are highly conserved as in the *cox1* gene near amino acid 240 where homologous introns have been found in the fungi, S. *cerevisiae, P. anserina, Spizellomyces punctatus, Rhizophus stolonifer,* the liverwort *Marchantia polymorpha,* and the plant *Peperomia polybotrya* (Paquin et al., Curr. Genet., 31:380-395 (1997)). Homologous introns also can be optional.

Intronic regions can include Groups I and II type introns as well as optional introns. Selected intronic regions are evaluated to determine their usefulness in differentiating between or among target organisms can be detected in nucleic acid of known organisms by a variety of methods. Such methods include analysis of nucleic acid from the target organism which can be detected directly by, for example, probe hybridization, cloning and sequencing or by analysis of amplified product from primer extension. Primer extension methods are preferred.

### Primer Extension and Signal Amplification Methods

The intron-amplifying primers are used to amplify products from target DNA in a primer extension reaction. A variety of primer extension reactions can be used with the present methods. Non PCR amplification methods include ligase chain reaction (LCR: Barany et al., PCR Meth. Applic., 1:15-16 (1991)), self-sustained sequence replication (SSR: Muller et al., Histochem. Cell Biol., 108:431-437 (1997)), also known as nucleic acid sequence-based amplification: NASBA) and its new derivative, cooperative amplification of templates by cross-hybridization (CATCH: Ehricht et al., Eur. J. Biochem., 243:358-364 (1997)), transcript-based amplification system (AMPLISCRIPT^{®}, Kaylx Biosciences, Nepean, Ontario Canada), replicatable RNA reporter systems based on the Q beta replicase, hybridization-based formats such as strand-displacement amplification (SDA: Becton-Dickinson, Franklin Lakes, NJ; Walker et al. Nucleic Acids Res., 20:1691-1696 (1992)), and chip-based microarrays such as Affymetrix GeneChip (Fodor et al., Nature, (Lond) 364:555-556 (1993)).

Signal amplification methods also can be used to enhance detectability such as with the use of compound probes (Fahrlander et al., Bio/Technology, 6:1165-1168 (1988)) or branched probes (Chiron Corp., Emeryville, CA; Urdea et al., Nucleic Acids Symp. Ser., 24:197-200 (1991)) as is well known in the art.

Primer extension by PCR is performed by combining one or more primers with the target nucleic acid and a PCR buffer containing a suitable nucleic acid polymerase. The mixture is thermocycled for a number of cycles, which is typically predetermined, sufficient for the formation of a PCR reaction product, thereby enriching the sample to be assayed for the presence, absence, size polymorphism or sequence polymorphism associated with a particular intronic region. Protocols for PCR are well known in the art (e.g., U.S. Pat. Nos. 4,683,192, 4,683,202, 4,800,159, and 4,965,188) and are available from a variety of sources (e.g., PCR Technology: Principles and Applications for DNA Amplification, H. Erlich, ed., Stockton Press, New York (1989); and PCR Protocols: A Guide to Methods and Applications, Innis et al., eds., Academic Press, San Diego, CA (1990)).

PCR is typically carried out by thermocycling, i.e., repeatedly increasing and decreasing the temperature of a PCR reaction admixture within a temperature range whose lower limit is about 30 degrees Celsius (30°C) to about 55°C, and whose upper limit is about 90°C to about 100°C. Increasing and decreasing the temperature can be continuous, but is preferably phasic with time periods of relative temperature stability at each of the temperatures favoring polynucleotide synthesis, denaturation and hybridization. Thus, the PCR mixture is heated to about 90-100°C for about 1 to 10 minutes, preferably from 1 to 4 minutes. After this heating period, the solution is allowed to cool to about 54°C, which is preferable for primer hybridization. The synthesis reaction may occur at room temperature up to a temperature above which the polymerase (inducing agent) no longer functions efficiently. Thus, for example, if Taq DNA polymerase is used as inducing agent, the temperature is generally about 70°C. The thermocycling is repeated until the desired amount of amplified product is produced.

A single intronic region-specific primer pair can be used in each amplification reaction. Alternatively, additional primers from other primers pairs can be included in the reaction. The primers are generally added in molar excess over template DNA. The conditions of the PCR are adjusted depending on a number of factors, including the degree of mismatch, the GC content of the primer, the length of the primer factors affecting PCR conditions, melting temperature of the primer, and product length and placement within the target sequence. Adjustments in the concentrations of the reaction components, especially magnesium concentration, can be used to enhance the conditions for PCR.

The PCR buffer contains the deoxyribonucleoside triphosphates (i.e., polynucleotide synthesis substrates) dATP, dCTP, dGTP, and dTTP and a polymerase, typically thermostable, all in amounts sufficient for the primer extension (i.e., polynucleotide synthesis) reaction. An exemplary PCR buffer comprises the following: 50 mM KC1; 10 mM Tris-HCl at pH 8.3; 1.5 mM MgCl₂ ; 0.001% (wt/vol) gelatin, 200 microMolar (µM) dATP, 200 µM dTTP, 200 µM dCTP, 200 µM dGTP, and 2.5 units Thermus aquaticus (Taq) DNA polymerase I (U.S. Pat. No. 4,889,818) per 100 microliters (µL) of buffer.

The inducing agent may be any compound or system which will function to accomplish the synthesis of primer extension products, including enzymes. Suitable enzymes for this purpose include, for example, E. coli DNA polymerase I, Klenow fragment ofE. coli DNA polymerase I, T4 DNA polymerase, other available DNA polymerases, reverse transcriptase, and other enzymes, such as heat-stable enzymes that facilitate combination of the nucleotides in the proper manner to form the primer extension products complementary to each nucleic acid strand. Generally, the synthesis will be initiated at the 3' end of each primer and proceed in the 5' direction along the template strand, until synthesis terminates, producing molecules of different lengths. There may be inducing agents, however, which initiate synthesis at the 5' end and proceed in the above direction, using the same process as described above. Intronic region-specific primers suitable for such inducing agents can be designed using the principles elaborated above for inducing agents that extend from the 3' end.

The PCR reaction can advantageously be used to incorporate into the product a preselected restriction site useful in later cloning and sequencing the amplified product. This can be accomplished by synthesizing the primer with the restriction site in the 5' end of the primer.

Nucleic acid from known organisms or products produced therefrom by primer extension reactions with intron-amplifying primers are analyzed to determine if the intronic region is present, absent, or varies by size (PDLP) and/or sequence in the DNA of target organisms. Primer-Defined Sequence Polymorphisms (PDSP) refer to differences in the sequences of amplified DNA in an intronic region of the amplified DNA sequence.

The amount of amplified nucleic acid product needed for analysis varies with the method chosen. Generally, about 1 to about 500 ng of amplified DNA product is required. As discussed above, a preferred primer extension method is PCR.

Fractionation of amplified products by size also is useful to evaluate differences in the length of the amplified intronic regions, referred to herein as a primer-defined length polymorphism (PDLP). PDLPs result, for example, from insertions or deletions in an intronic region. To detect PDLPs, the amplified DNA sequence is located in a region containing insertions or deletions of a size that is detectable by the chosen method. The amplified DNA sequence should be of a size that is readily resolved by the method chosen.

The presence or absence of the intronic regions in a target DNA is typically determined by analyzing the amplified nucleic acid products of the primer extension by size using standard methods, for example, agarose gel electrophoresis, polyacrylamide gel electrophoresis, capillary electrophoresis, pulsed field electrophoresis, and denatured gradient gel electrophoresis (DGGE). Non size based method include, for example, single stranded conformational polymorphism (SSCP). All of these methods are well known in the art (e.g., Sambrook et al., *supra,* (1989) (6.3-6.6); Nucleic Acid Electrophoresis (D. Teitz, ed.), Springer Verlag, New York (1998).

DNA electrophoresis involves separation, usually in a supporting medium, by size and charge under the influence of an applied electric field. Gel sheets or slabs, e.g., agarose, agarose-acrylamide or polyacrylamide, are typically used for nucleotide sizing gels. Nucleic acid products of about 20 bp to > 10,000 bases in length can be optimally resolved in the above electrophoretic methods in combination with different types of agarose. Nucleotide sequences which differ in length by as few as 3 nucleotides (nt), preferably 25 to 50 nt, can be distinguished by electrophoresis. Sequences as long as 800 to 2,000 nt, which differ by at least about 50 nt, also are readily distinguishable.

Preparation and staining of analytical nucleic acid electrophoretic gels is well known. For example, a 3% Nusieve 1% agarose gel which is stained using ethidium bromide is described in Boerwinkle et al., Proc. Natl. Acad. Sci. (USA), 86:212-216 (1989). Detection of DNA in polyacrylamide gels using silver stain is described in Goldman et al., Electrophoresis, 3:24-26 (1982); Marshall, Electrophoresis, 4:269-272 (1983); Tegelstrom, Electrophoresis, 7:226-229 (1987); and Allen et al., BioTechniques, 7:736-744 (1989). Nucleic acid also can be labeled with an isotope such as ³²P and detected after gel electrophoresis by autoradiography.

Size markers can be run on the same gel to permit estimation of the size of the amplified products or their restriction fragments. Comparison to one or more control sample(s) can be made in addition to or in place of the use of size markers. The size markers or control samples are usually run in one or both the lanes at the edge of the gel, and preferably, also in at least one central lane. In carrying out the electrophoresis, the DNA fragments are loaded onto one end of the gel slab (commonly called the "origin") and the fragments separated by electrically facilitated transport through the gel, with the shortest fragment electrophoresing from the origin towards the other (anode) end of the slab at the fastest rate. An agarose slab gel is typically electrophoresed using about 5-15 volts/cm of gel for 30 to 45 minutes. A polyacrylamide slab gel is typically electrophoresed using about 200 to 1,200 volts for 45 to 60 minutes.

Tables 1 and 2 in Example 3, summarize the results of size analysis of PCR amplified products by agarose gel electrophoresis. In this example, intronic region-specific primer pairs for detecting multiple intronic regions of the *cox* 1 gene were used to amplify product in template DNA from several species of the genus *Candida* and other fungi. Intron polymorphisms were identified between members of the genus *Candida* as differences in size as well as the absence of the intron.

In cases where hybridization assays of multiple target organism genomes are desired to be performed simultaneously using the same intronic region-specific probes, it would be convenient to perform such hybridizations in an array format. Such assay formats and minaturizations thereof, i.e. microchip assays, are well known in the literature and could easily be adapted for the assays described herein. For example, see PCT WO 00/03037, which describes screening arrays of nucleotides using specific probes. After compilation of the intronic region profile for a given taxonomic group, the nucleotide sequences corresponding to the intronic regions of the different organisms belonging to the taxonomic group can be used in a microarray format on a microchip to perform simultaneous hybridization studies with various probes or sequences from unknown organisms.

Alternatively, such assay formats can be designed for use to study hybridization of an array of intronic region-specific sequences with a single target organism genome, or an array of the protein products derived from the translation of intronic sequences of unknown organisms, or an array of antibodies to such protein products, or combinations thereof in two-dimensional arrays. Such hybridization microarray assays can easily be performed using a variety of known microchip assay formats and techniques.

### Sequencing Analysis

Analysis of nucleic acid from known target organisms or products produced therefrom by primer extension as described herein also can include analysis of the sequence of the amplified intronic region including an adjoining exon of the target template DNA. Intronic region sequence as well as intronic region size can be determined by cloning and sequencing the intronic region. For example, amplified products such as from a PCR can be directly cloned by a variety of methods well known in the art (e.g., Ausubel et al., Molecular cloning of PCR products, in: Short Protocols in Molecular Biology, 3rd Ed. John Wiley & Sons, Inc., New York, pp. 15-32 (1997)). Cloning of amplified products can be accomplished using "sticky ends" such as the TA cloning method or by "blunt end" cloning approaches. Alternatively, intronic region-specific primers can be designed with endonuclease restriction sites at the 5' end of the primer which are designed for cutting and insertion into a specified cloning vector. Kits are commercially available for cloning amplified products such as produced in a PCR (e.g., Invitrogen, Inc., San Diego, CA). Cloned intronic regions of the *cox*1 mitochondrial gene from fungi are provided in Example 8.

Methods for sequencing genes are well known, including the Sanger dideoxy mediated chain-termination approach and the Maxam-Gilbert chemical degradation approach. These and other nucleic acid sequencing methods are described, for example, in Sambrook et al., *supra*, (1989) (chapter 13). Nucleic acid sequencing can be automated using a number of commercially available instruments.

Amplified products also can be directly sequenced without cloning the product (e.g., Sambrook et al., *supra*, (1989) (14.22-14.29)). Amplified products that have been purified, for example, by gel electrophoresis, are suitable for direct sequencing (id.).

Differences in the sequence of amplified products produced by primer extension with intronic region-specific primers also can be analyzed by RFLP. Direct sequencing is preferred over RFLP. However, RFLP analysis of amplified products from different DNA target templates can provide a screening tool for detecting sequence differences of similar sized products.

Restriction enzymes for performing RFLP are available commercially from a number of sources including Sigma Chemical Co. (St. Louis, MO), Bethesda Research Labs (Bethesda, MD), Boehringer-Manheim (Indianapolis, IN) and Pharmacia & Upjohn (Bridgewater, NJ). Endonucleases are chosen so that by using a plurality of digests of the amplified sequence, preferably fewer than five, more preferably two or three digests, the amplified products can be distinguished.

Intronic region-specific primers that are designed from aligned sequences are referred to herein are "first generation" primers because they are complementary to a consensus sequence. In contrast, when sequence information is obtained for amplified products, "second generation" intronic region-specific primers can be designed that are complementary to a specific primer site target sequence. Such second generation primers have increased specificity for particular organisms and can be designed to yield sizes of amplified intronic regions that are easier to detect. The products of the second generation primers may be detected as nucleic acids using methods described above. Second generation primers are preferred for the method of detecting an organism in a sample as discussed below.

### Protein Detection Methods

Particular intronic regions that comprise all or a portion of an open reading frame (ORF) that encodes a protein (e.g., an enzyme) can be detected for their presence or absence in nucleic acid from known organisms by using antibodies specific for encoded protein or detection based on the enzymatic activity of the protein. Such enzymatic activity can include, for example, endonuclease, maturase or reverse transcriptase activity.

The expression of such an intronic region encoded protein ("IREP") by the organism, which is detected by an anti-IREP antibody, can be used to identify the organism. Using this approach, one can determine if the organism from which the protein is derived is living by incubating the sample under suitable conditions with one or more labeled amino acids precursors and determining if the label is associated with the intronic region protein.

Whether an intronic region encodes a protein can be detected using software programs that detect open reading frames based on all possible start and stop codons (e.g., MacVector v. 5.0.2). Example 8 discloses consensus sequences of five *cox*1 fungal mitochondrial introns, four of which contain an open reading frame. The sequence of the encoded ORF for the cloned *cox*1 genes are provided in Example 8.

Monoclonal antibodies or polyclonal antisera raised against antigenic epitopes of the IREP are useful if the antigenic epitopes they detect differentiate between or among different taxonomic groupings of organisms. Binding of the anti-IREP antibody to the antigenic epitopes of the organism can be determined by methods well known in the art, including SDS-PAGE, Western Blotting, isoelectric focusing, 2-D gels, immunoprecipitation, epitope tagging, radioimmunoassay, enzyme-linked immunoadsorbent assay (ELISA), fluorescence and the like.

An anti-IREP antibody is used in its broadest sense to include polyclonal and monoclonal antibodies, as well as polypeptide fragments of antibodies that retain a specific binding affinity for its target antigen of at least about 1x10⁵ M⁻¹. One skilled in the art would know that antibody fragments such as Fab, F(ab')₂ and Fv fragments can retain specific binding activity for their target antigen and, thus, are included within the definition of an antibody herein. In addition, the term "antibody" as used herein includes naturally occurring antibodies as well as non-naturally occurring antibodies such as domain-deleted antibodies (Morrison et al., WO 89/07142) or single chain Fv (Ladner et al., U.S. Pat. No. 5,250,203). Such non-naturally occurring antibodies can be constructed using solid phase peptide synthesis, can be produced recombinantly or can be obtained, for example, by screening combinatorial libraries consisting of variable heavy chains and variable light chains as described by Huse et al., Science, 246:1275-1281 (1989).

Antibodies to IREPs can be prepared using a substantially purified IREP, or a fragment thereof, which can be obtained from natural sources or produced by recombinant DNA methods or chemical synthesis. For example, recombinant DNA methods can be used to express the intronic ORF sequence alone or as a fusion protein, the latter facilitating purification of the antigen and enhancing its immunogenicity.

If the IREP is not sufficiently immunogenic, it can be coupled to an immunogenic carrier molecule chemically or expressed as a fusion protein with such immunogenic carriers as bovine serum albumin or keyhole limpet hemocyanin (KLH). Various other carrier molecules and methods for coupling a non-immunogenic peptide to a carrier molecule are well known in the art (e.g., Harlow and Lane, "Antibodies: A Laboratory Manual," Cold Spring Harbor Laboratory Press (1988)).

Antisera containing polyclonal antibodies reactive with antigenic epitopes of the IREP can be raised in rabbits, goats or other animals. The resulting antiserum can be processed by purification of an IgG antibody fraction using protein A-Sepharose chromatography and, if desired, can be further purified by affinity chromatography using, for example, Sepharose conjugated with a peptide antigen. The ability of polyclonal antibodies to specifically bind to a given molecule can be manipulated, for example, by dilution or by adsorption to remove crossreacting antibodies to a non-target molecule. Methods to manipulate the specificity of polyclonal antibodies are well known to those in the art (e.g., Harlow and Lane, *supra,* (1988)).

A monoclonal antibody specific for the IREP can be produced using known methods (Harlow and Lane, *supra,* (1988)). Essentially, spleen cells from a mouse or rat immunized as discussed above are fused to an appropriate myeloma cell line such as SP2/0 myeloma cells to produce hybridoma cells. Cloned hybridoma cell lines can be screened using a labeled IREP to identify clones that secrete an appropriate monoclonal antibody. An IREP can be labeled as described below. A hybridoma that expresses an antibody having a desirable specificity and affinity can be isolated and utilized as a continuous source of monoclonal antibodies. Methods for identifying an anti-IREP antibody having an appropriate specificity and affinity and, therefore, useful in the invention are known in the art and include, for example, enzyme-linked immunoadsorbence assays, radioimmunoassays, precipitin assays and immunohistochemical analyses (e.g., Harlow and Lane, *supra,* (1988) (chapter 14)).

An anti-IREP antibody can be characterized by its ability to bind specifically to the organisms that express the particular IREP. Because organelles such as mitochondria are inside cells, the cells may need to be permeabilized to allow access of the antibody to the organelle. Methods to permeabilize cells are such as by treating with detergents are well known in the art (e.g., Harlow and Lane, *supra*, (1988)). Alternatively, a sample containing the organism can be subjected to protein purification methods to obtain a cell-free protein fraction suitable for antibody binding.

An anti-IREP antibody of the invention can be used to purify IREP in a sample. For example, such antibodies can be attached to a solid substrate such as a resin and can be used to affinity purify the IREP. In addition, the anti-IREP antibody can be used to identify the presence of the IREP in a sample. In this case, the antibody can be labeled with a detectable moiety such as a radioisotope, an enzyme, a fluorochrome or biotin. An anti-IREP antibody can be detectably labeled using methods well known in the art (e.g., Harlow and Lane, *supra,* (1988) (chapter 9)). Following contact of a labeled anti-IREP antibody with a sample, specifically bound labeled antibody can be identified by detecting the moiety.

The binding of an anti-IREP antibody to the IREP also can be determined using immunological binding reagents. As used herein, an immunological binding reagent includes any type ofbiomolecule that is useful to detect an antibody molecule. An immunological binding reagent can include a labeled second antibody. A second antibody generally will be specific for the particular class of the first antibody. For example, if an anti-IREP antibody (i.e., a first antibody) is of the IgG class, a second antibody will be an anti-IgG antibody. Such second antibodies are readily available from commercial sources. The second antibody can be labeled using a detectable moiety as described above. When a sample is labeled using a second antibody, the sample is first contacted with a first antibody (i.e., anti-IREP antibody), then the sample is contacted with the labeled second antibody, which specifically binds to the first antibody and results in a labeled sample. Alternatively, a labeled second antibody can be one that reacts with a chemical moiety, for example biotin or a hapten that has been conjugated to the first antibody (e.g., Harlow and Lane, *supra,* (1988) (chapter 9)). Immunological binding agents also can include avidin or streptavidin when the anti-IREP antibody is labeled with biotin.

Principally, all conventional immunoassays are suitable for the detection of IREPs. Direct binding as discussed above or competitive tests can be used. In a competitive test, the antibody can be incubated with a sample and with the IREP or a fragment thereof (produced as described herein) both simultaneously or sequentially. The IREP from the sample preferably competes with the added IREP (hapten) of the invention for the binding to the antibody, so that the binding of the antibody to the hapten in accordance with the invention is a measure for the quantity of antigen contained in the sample. In a heterogeneous competitive immunoassay where the liquid phase is separated from the solid phase, both the antibody or the peptide can be labeled or bound to a solid phase. The exact amount of antigen contained in the sample can then be determined in a conventional manner by comparison with a standard treated in the same manner.

All competitive test formats that are known to the expert can be used for the detection. The detection can be carried out, for example, using the turbidimetric inhibition immunoassay (TINIA) or a latex particle immunoassay (LPIA). When a TINIA is used, the peptide or peptide derivative of the invention is bound to a carrier such as dextran (EP-A-0 545 350). This polyhapten competes with the analyte contained in the sample for the binding to the antibody. The formed complex can be determined either turbidimetrically or nephelometrically. When an LPIA is employed, particles, preferably latex particles, are coated with the peptides of the invention and mixed with the antibody of the invention and the sample. When an analyte is present in the sample, agglutination is reduced.

Enzyme immunoassays (Wisdom, Clin. Chem., 22(8):1243-1255 (1976), and Oellerich, J. Clin. Chem. Clin. Biochem., 18:197-208 (1980)), fluorescence polarization immunoassays (FPIA) (Dandliker et al., J. Exp. Med., 122:1029 (1965)), enzyme-multiplied immunoassay technology (EMIT) (Rubenstein, Biochem. Biophys. Res. Comm., 47:846-851 (1972)) or the CEDIA technology (Henderson et al., Clin. Chem., 32:1637-41 (1986)) also are suitable immunological based assays for detection of intronic IREPs.

If useful, organisms can be identified using both nucleic acid based detection of an intronic region and the immunological approach which uses anti-IREP antibodies to identify intronic regions encoding a protein.

### E. Methods of Identifying an Organism in a Sample

The present description also discloses methods of identifying the presence of a specific organism in a sample, comprising detecting the presence or absence of one or more intronic regions in the nucleic acid of the organism that are characteristic of the organism. The method of detection can be used to diagnose the presence of virtually any organism that contains DNA including fungi, protozoans and other members of the animal kingdom and members of the plant kingdom. Fungi suitable for detection by intron polymorphism analysis include members of the genus of *Candida, Aspergillus, Coccidiodes, Cryptococcus, Histoplasma, Blastomyces, Cladosporium* for clinical applications, and *Aspergillus, Fusarium, Tilletia, Puccinia, Septoria, Botrytis, Pyrenophora,* and *Gaeumannomyces* for nonclinical applications.

An organism can be identified by detecting the presence or absence of one or more intronic regions. The number of intronic regions that need to be evaluated for identifying a particular organism depends on a number of factors, including the uniqueness of a particular intronic region and the potential for related species of organisms to be present in the sample. Generally, a lesser number of introns will need to be evaluated if the goal is to determine a broad classification of the infecting organisms, such as family or genus. In contrast, a larger number of introns generally will need to be analyzed if the goal is to identify a single species of organism or distinguish between races or strains of a single species. By evaluating a sufficient number of intronic regions, the identity of the organism can be established with confidence and significant false negative and false positive results avoided.

In addition, an organism can be identified by detecting intronic regions from more than one source. Thus, intronic regions from different genes can be detected and these genes can be from nuclear DNA or organellar DNA.

Detecting the presence or absence of intronic regions can be accomplished by a variety of methods well known in the art for detecting nucleic acids. These include, for example, primer extension reactions, separation of amplified products by molecular weight, nucleotide sequencing, RFLP or hybridization with a specific nucleic acid probe.

### Detection by Primer Extension

The approaches described above for identifying intronic regions that can differentiate between or among taxonomic groups by primer extension also are generally applicable for identifying a specific organism in a sample. For example, the strategy for designing intronic region-specific primers are similar for both identification of intronic regions and for detecting such regions for organism identification. Both first generation and second generation intronic region-specific pairs are useful for organism identification Second generation primers, however, are preferred because they are complementary and, therefore, can be used in primer extension reactions under high stringency conditions. Also, PCR is the preferred choice of primer extension reaction.

The amplifying primer sites are in the exon sequence immediately adjacent to the intron insertion site of the gene. In this case, primer extension will result in a very small sized product (about the combined length of the two primers or so) if the template DNA lacks the intronic region and potentially a much lager product if the template DNA contains the intronic region. In another embodiment, the amplifying primers are located farther from the intron insertion site, for example in a non-flanking exon. In this case, primer extension will generate a larger product than in the case when the primer sites directly flank the intronic region. In yet another embodiment, the intronic region-specific primer sites are located sufficiently far apart so that they span more than one intron insertion site. In this way, amplification by primer extension can generate a product that contains multiple intronic regions.

The intronic region-specific primer sites are preferably located in conservative regions of the gene. In one embodiment, the intronic region-specific primer sites are located in a conserved region of the intron or in an adjacent, upstream and/or downstream exon sequence. In another embodiment, the intronic region-specific primer sites are located in an upstream or downstream intron.

### Detection bv Probe Hybridization

The presence or absence of a particular intronic region can be determined by standard hybridization with a nucleic acid probe. The probe is preferably a second generation intronic region-specific primer or any other polynucleotide that is complementary to the target sequence. Such probes can be prepared by synthesis or be obtained from nucleic acid vectors containing the probe sequence.

Amplified nucleic acid sequences derived from primer extension with the intronic region-specific primers also can be used as a probe for detecting the presence or absence of an intronic region.

The probe can be labeled with a detectable atom, radical or ligand using any of a variety of known labeling techniques. For example, the probe can be labeled with ³²P by nick translation with an alpha-³²P-dNTP (Rigby et al., J. Mol. Biol., 113:237 (1977)) or labeled with an enzyme, such as horseradish peroxidase and binding detected by production of a visible substrate. Methods of preparing and labeling probes are well known in the art (e.g., Sambrook et al., *supra,* (1989) (11.21-11.44)).

Where the nucleic acid containing a target sequence is in a double stranded (ds) form, it is preferred to first denature the dsDNA, as by heating or alkali treatment, prior to conducting the hybridization reaction. The denaturation of the dsDNA can be carried out before or after adding the probe.

The amount of nucleic acid probe used in the hybridization reaction is generally well known and is typically expressed in terms of molar ratios between the probe and the target. Preferred ratios contain equimolar amounts of the target sequence and the probe although it is well known that deviations from equal molarity will produce hybridization reaction products at lower efficiency. Thus, although ratios can be used where one component is included at 100-fold molar excess relative to the other component, excesses of less than 50-fold, preferably less than 10-fold, and more preferably less than two-fold are desirable in practicing the invention.

### Inclusion of Controls for Detecting Organisms

The present methods of detecting an organism in a sample also can include controls to avoid false negative and false positive results. False-positive results are avoided if the detection method used is highly selective. In primer extension reactions, it is recommended to include internal controls and to confirm any new or unusual results by an independent amplification reaction (Ieven, et al., Clin. Microbiol. Rev., 10:242-256 (1997)). False-positive results also can be prevented by removing sources of contamination in sample handling or carryover from previous experiments.

The detection method disclosed herein avoids many of these difficulties because a collection of intronic region-specific primers is used to yield independent products. For example, an unexpected novel combination of previously known products or a set of previously unknown products would signal a possible false positive that could then be confirmed in an independent DNA sample with other primer pairs.

False-negative results occur when a detection method lacks sensitivity or is subject to a sampling error (e.g., when a PCR is performed on an aliquot that lacks template). When detecting pathogens directly in a sample (e.g., a field or clinical specimen), the lack of sensitivity can be due to the presence of some unknown inhibitor of the primer extension reaction. A polynucleotide whose sequence is derived from the diagnostic primer sequences, along with the diagnostic primers can be used in primer extension to yield an internal control product that is easily distinguished from the expected product by its larger size. The internal control product, when co-amplified with a titration of known amounts of target DNA, also can be used to quantify the amount of template present in the sample (e.g., Honeycutt et al., Anal. Biochem., 248:303-306 (1997)).

The sensitivity of the method to detect an intronic region can be increased with the use of second generation primers. Second generation primers are based on the intronic sequence and exonic flanking sequences determined with first generation primers. Sensitivity can be increased by selecting primer sites for the second generation primers that yield a small product in the PCR when target template is present. The second-generation primers are complementary to the target nucleic acid and, therefore, can be used under conditions of high stringency in the PCR. Under such conditions, the small PCR product can out-compete larger arbitrary PCR products that might arise from the host genome, thus increasing the sensitivity of the detection method. Small products also are amenable to existing automated TAQMAN^{®} (Perkin-Elmer, Foster City, CA: Holland et al., Proc. Natl. Acad. Sci (USA), 88:7276-7280 (1991) as well as non-PCR amplification technologies such as NASBA, LCR, SDA and TMA.

### Detection by Immunological Methods

The identity of a particular organism in a sample can be determined by detecting the presence or absence of particular intronic regions that encode IREPs. Detection of such IREPs, which indirectly indicate the presence of the encoding intronic region, can be accomplished by immunological based assays using anti-IREPs produced as described above. Principally, all conventional immunoassays are suitable for the detection of IREPs including direct binding or competitive tests as discussed above.

### F. Kits for Detecting Intronic Regions

The present description also discloses kits that incorporate the components described. Kits comprise one or more of the reagents used in the above described methods and may also include other materials that would make the invention a part of other procedures including adaptation to multi-well technologies. The items comprising the kit may be supplied in separate vials or may mixed together, where appropriate.

A kit can comprise at least one intron-amplifying-specific primer pair in a suitable container. Preferably the kit contains two or more intronic region-specific primer pairs. In another embodiment, the primer pairs are useful for different intronic regions of different genes and are in separate containers. In another embodiment, the primer pairs are specific for intronic regions of a single gene. Primer pairs can be combined provided there is no interference when used together in amplification or hybridization methods. If necessary, individual primers of each primer pair can be kept in separate vials.

The kit additionally can include in internal amplification control that contains a primer site for the intronic region-specific primers. Additional reagents such as amplification buffer, digestion buffer, a DNA polymerase and nucleoside triphosphates also can be included in the kit.

The primers can be provided in a small volume (e.g., 100 µl) of a suitable solution such as sterile water or Tris buffer and can be frozen. Alternatively, the primers can be air-dried. In another embodiment, a kit comprises, in separate containers, an intronic region-specific probe and solutions for performing hybridization.

Kits are disclosed for immunological based detection of intronic regions that are expressed by the organism. Such kits can include one or more specific antibody, and an immunological binding reagent to detect binding of the specific antibody. These reagents are preferably provided in separate containers.

### Therapeutic Methods

As described above, methods are provided herein for identifying intronic regions that are specific for taxonomic groupings of organisms. Also described above are methods for characterizing taxonomic groupings of organisms based on detection and differentiation of the intronic region encoded proteins ("IREPs"). A further extension of this technological platform, which is described below, is the targeting of such intronic regions and associated IREPs for therapeutic purposes. Just like the intronic region sequences are known to be specific for taxonomic groupings of organisms, so are their corresponding IREPs. Accordingly, therapeutic applications based on intronic region specificity provides a taxonomic group-specific approach. Since Group I and Group II introns are not known to exist in mammals, but are commonly found in fungi and other eukaryotic microorganisms and plants, these applications are ideal for specifically targeting mammalian and plant pathogenic microorganisms, as well as the plants themselves via chloroplast-encoded introns.

The present invention is based on the realization that primarily targeting non-splicosome-mediated, non-autocatalytic post-transcriptional processing of pre-RNA, referred to herein as "IREP-mediated" RNA processing, provides for a useful therapeutic approach. Most Group I and Group II organellar intron-associated post-transcriptional RNA processing is mediated by IREPs with RNA sequence-specific activities, such as maturase, reverse transcriptase and endonuclease activities. Although reverse transcriptase and endonuclease activities are well characterized, the details of the mechanism of action of the organellar maturases are not completely understood. Although not wishing to be bound by any particular theory, it is believed that maturases function by stabilizing RNA conformation during cleavage ((Nucl. Acid Res. 25:3379-3388 (1997)). According to this theory, a maturase binds the intron that encodes for that maturase, and this binding changes and stabilizes the conformation of the pre-RNA, secondary structure in a manner that promotes autocatalysis.

The Group I introns are divided into four different classes of proteins, depending on their presence of evolutionarily conserved regions, as follows:
1. LAGLI-DADG MOTIF
2. HIS-CYS BOX MOTIF
3. GIY-YIG MOTIF
4. HNH MOTIF
(Cell. Mol. Life Sci. 55: 1304-1326 (1999))

The therapeutic agents can modulate activity of members of the LAGLI-DADG class of proteins, which includes the maturases, which is further defined as proteins having at least one LAGLI-DADG amino acid motif, as well as homologues thereof having at least 70 and preferably 85% homology as further described by Dalgaard et al. (Nucleic Acids Res. 35: 4626-4638 (1997).) It should also be mentioned that, although the methods and compositions described herein are considered to have modes of action which are primarily "non-autocatalytic" and-"non-splicosome mediated", this terminology intends only that their primary mode of action is not dependent on either of these mechanisms.

Although in some instances the maturase-mediated RNA cleavage site is also capable of self-cleavage, it does so at much higher magnesium concentration Accordingly, one can identify if a compound is inhibiting protein-mediated or self-splicing by adjusting the Mg2+concentration. This is in contrast to splicing of nuclear-encoded introns, which requires a number of proteins, small ribonucleoprotein factors and pre-RNA sequence signals, collectively forming the splicing machinery known as "splicesomes", that function during the maturation process of pre-RNA to mature RNA in the nucleus (PCT WO 00/65780). Thus for one of skill in the art, maturase-mediated splicing of organellar Group I and Group II introns is easily distinguished from the splicing of other introns.

Accordingly, techniques exist to differentiate autocalalytic and splicosome-mediated RNA cleavage from IREP-mediated cleavage, particularly in terms of the effect of proposed antimicrobial agents on the specific activities of IREPs, such as maturase and homing endonuclease activities. By way of example, what follows is a model system for maturase function.

A simplified overview of the series of events in maturase-mediated splicing is shown in Figure 3 as follows: Say, for example, a gene contains two introns, i1 and i2, which interrupt the genes' three exons, e1, e2, and e3, respectively. Each of the two introns contains a single open-reading frame (ORF) that is continuous with its immediately preceding 5' exon. Each of the open reading frames encode a maturase (ORFm). During transcription, the entire gene sequence is transcribed into a pre-mRNA molecule "etile2i2e3". Translation is initiated and proceeds until the ribosomes reach the terminator codon of the open-reading frame encoded in i1. The result is a truncated "eli1ORFm" protein. Because the eli1ORFm protein has trans-acting maturase activity, the maturase recognizes and binds another "eli1e2i2e3" pre-mRNA molecule and i1 is cleaved and excised from the molecule; el ligates to e2i2e3 generating a partially processed "ele2i2e3" pre-mRNA molecule. Translation initiates on the "e1e2i2e3" pre-mRNA molecule and proceeds until the ribosomes reach the terminator codon of the open-reading frame encoded in i2. The ribosomal machinery truncates the "e1e2i2ORFm" protein. Again, i2ORFm has maturase activity and i2 is excised out of "ele2i2e3" pre-mRNA molecules; "e1e2" and "e3" ligate generating a completely processed mRNA molecule. Translation is intiated and completed on the mature "e1e2e3" mRNA; the resulting protein may or may not be further processed or modified after translation is completed. Post-translational processing is well known and is sometimes intron-related. For example, in some organisms, notably T-even bacteriophage, intron-encoded proteins referred to as inteins, have peptidase activity and cleave post-translationally other proteins (see for example U.S. Pat. No. 5,795,731).

Partially processed pre-mRNA molecules containing introns, such as e1e2i2e3, have measurable half-lives as detected on Northern blots of yeast mitochondria from cultures grown on pyruvate (Jacq., et al., EMBO J. 3:1567-1572 (1984)). Maturases may be required only in catalytic amounts, however an abundance of maturases does not appear to be lethal (Lázowska, et al., Cell 22:333-348 (1980).)

It will be well understood by one of skill in the art that high-throughput screening assays can be adapted for use in screening virtually any of the activities commonly associated with IREPs. Intron splicing in many different taxonomic groups of organisms has been described. Many RNAs from lower organisms such as bacteria are characterized as being "self-splicing" (Nucleic Acids Research 24(24):5051-5053 (1996).) As described therein, screening assays have been developed to identify small molecules that are effective anti-microbial agents because they interfere with RNA self-splicing. However, as described herein, the present invention relates to the design and identification of compounds whose primary mode of action is modulation of IREP mediated RNA processing found in organisms such as fungi and other organelle-containing organisms.

Candidate compounds are identified from, for example, a small molecule combinatorial library by including the compound in the growth medium. Without regard to the specific nature of any particular compound, outcomes anticipated in this assay are as shown in Table 1 of Example 9: yeast fail to grow; yeast grow and do not express a reporter gene product, such as green fluorescent protein (gfp); yeast grow and do express gfp. Compounds that yield the first outcome are lethal to yeast and are less desirable as therapeutic candidates because their mode of action may disrupt a target that is common in the target organism and its host. Compounds that yield the second outcome disrupt maturase activity and are candidates for therapeutic uses because growth of the organism is specifically inhibited. Finally, compounds that yield the third outcome fail to disrupt maturase activity, do not inhibit the organism's growth and so are not candidates for therapeutic uses.

In addition to screening small molecules, also contemplated by the present invention is a molecular biology approach to maturase inhibition, for example using an antisense nucleic acid on nucleic acid-like molecule that binds to the maturase recognition site in the RNA to prevent its binding. Likewise, antibodies can be used as described in the above diagnostic section to inhibit maturase function.

It will be well understood by one of skill in the art that high-throughput screening assays can be adapted for use in screening virtually any IREP associated activities. Since such activities are well characterized, one can readily design microassays to assess function in a variety of different assay formats.

Protocols for formulating and administering antimicrobial agents to host organisms are well known in the art. As would also be known, such protocols depend on the nature of the agent itself, and in particular on its chemical, physical and biological properties.

In order to determine appropriate therapeutic protocols, one can easily use animal models and extrapolate to other mammalian hosts. For example, if the microbial pathogen to be treated is candida albicans, a group of mice can be innoculated with the pathogen and their mortality and/or pathogenic morphology studied over time after administration of various doses of agents of interest. See, e.g. U.S. Pat. No. 6,156,730, which discloses animal models for administration of peptides with antifungal properties.

In general, the agents that are identified according to the methods described herein are employed in combination with a suitable phamaceutically acceptable, preferably sterile carrier, such as saline, dextrose, water, glycerol and the like. Such compositions are administered according to their intended use as, e.g., injectables, oral medications, topical sprays, creams, aerosols and impregnated wound dressings. For other examples, see PCT WO 60/67580.

### EXAMPLES

### Example 1

### Consensus Alignment of Mitochondrial Gene Homologs

This example shows the selection and alignment of mitochondrial gene homologs of the cytochrome oxidase subunit 1 (*cox*1) gene for identifying introns suitable for discrimination between species of the fungal genus, *Candida. Cox*1 gene sequences are available representing a larger number of accessions than other mitochondrial genes and the gene is common to all fungi.

The *cox*1 sequences of fifteen accessions were downloaded from GOBASE, an Organelle Genome Database (http://megasun.bch.umontreal.ca/gobase/) as individual exon sequence files, and then merged. Of the fifteen accessions, thirteen are *Ascomycetes*, one is a *Basidiomycete*, and one is a *Chytridiomycete.* The *cox*1 gene of eleven of these accessions is interrupted by at least one intron with the number of introns varying between one and sixteen. The exon sequences were aligned using MAP (Multiple Alignment Program).

The position of intron insertion sites in *cox*1 was manually located on the exon alignments of the accessions containing introns. Figure 1 schematically depicts the location of a total of 38 unique intron insertions sites which are distributed along approximately 1400 of the 1800 bases in the exon consensus alignment in the *cox*1 gene. Primer pairs were derived that flanked four different multiple intron-containing regions as depicted in Figure 1. The large number of introns in *cox*1 provides an abundance of potential "intron amplifying" primer targets.

### Example 2

### Designing Intronic Region-Specific Primer Pairs

In this example, four multiple intronic region primer pairs were designed that collectively flank a total of 18 of the intron insertion sites in the *cox*1 gene as depicted in Figure 1. The primers were derived from the most conserved regions within the gene and contained the majority base of the alignment at each position. The 3'-most base of the primer was situated either in the first or second position within the reading frame so that the 3'-most base was not in wobble position of a codon. The primer was chosen so that there is no redundant base in the 3'-most position of the primer. In this manner, the primers had the greatest utility for testing a wide taxonomic group of accessions. The primers contained 20 to 23 nt with a GC content of 50% and similar predicted melting temperatures.

A total of 28 intronic region region-specific primers were designed based on the *Cox*1, *Cox*2 and *Nad*1 mitochondrial sequences. Sixteen primers were designed for *Cox*1 intronic regions (SEQ ID Nos. 1-16), eight primers were designed for *Cox*2 (SEQ ID Nos. 17-24) and four primers were designed for *Nad*1 intronic sequences. The primers are listed in the table below.

**Table 1. Intronic Region-Specific Primers for Fungal Mitochondrial Introns**

| Probe Designation | Nucleotide Sequence (5'-3') |
|---|---|
| cox1B4483 (SEQ ID NO: 1) | GCCTCCCTCATTATTATTATT |
| cox1B4803 (SEQ ID NO: 2) | CATTAGTTGAAAATGGAGCTG |
| cox1B5665 (SEQ ID NO: 3) | AATCTACGGTACCTCCAGAATG |
| cox1B5855 (SEQ ID NO: 4) | CTGTAAACTAAATATAGCTAAAT |
| cox1B8975 (SEQ ID NO: 5) | CTTACTATCCCAAATCCTGGT |
| cox1B7483 (SEQ ID NO: 6) | CATTACAATGTTATTAACTGATAGA |
| cox1B8103 (SEQ ID NO: 7) | GAGATCCTATTTTATATCAAC |
| cox1B9295 (SEQ ID NO: 8) | TAGGTTTACCTGAAAATGTTGA |
| cox1B10173 (SEQ ID NO: 9) | TAGGTTTAGATGTAGATACGAGA |
| cox1B10623 (SEQ ID NO: 10) | TGGTTATAGCTGTTCCAACTG |
| cox1B11255 (SEQ ID NO: 11) | CTACCACCATATAATGTAG |
| cox1B11655 (SEQ ID NO: 12) | ACCTAATACAAATAATAATGGT |
| cox1B11213 (SEQ ID NO: 13) | GGTAGTTTAAGATATAATACAC |
| cox1B11703 (SEQ ID NO: 14) | TGACTTTATTCACTATAGGAG |
| cox1B12225 (SEQ ID NO: 15) | AGAAGCATTAGATAATACTAC |
| cox1B12965 (SEQ ID NO: 16) | TACAGCTCCCATAGATAATACA |
| cox2B5433 (SEQ ID NO: 17) | ACCTACAGGAGTGCATATTCGA |
| cox2B5963 (SEQ ID NO: 18) | ACTTCGCCGTACCATCATTAGG |
| cox2B6805 (SEQ ID NO: 19) | CTTCACGTTTGATTAGTACTGA |
| cox2B7055 (SEQ ID NO: 20) | TCTCAACATTGTCCGTAGAATAC |
| cox2B6573 (SEQ ID NO: 21) | CATCAGTACTAATCAAACGAG |
| cox2B6813 (SEQ ID NO: 22) | GAGTATTCTACGGACAATGT |
| cox2B7545 (SEQ ID NO: 23) | TGATTCTACGGCAATAGGCA |
| cox2B7955 (SEQ ID NO: 24) | GATTGTGAGTCAAGCCAGCTT |
| nad1B998 (SEQ ID NO: 25) | ATGTTCTGTTTCTTATTCGTATG |
| nad1B10273 (SEQ ID NO: 26) | TGCTACTCTACCTCGACTAC |
| nad1B10725 (SEQ ID NO: 27) | ACAGAAGACCATTAACTGATC |
| nad1B11075 (SEQ ID NO: 28) | ACTAGAGCGATAGCAATAG |

The primers in Table 1 can be used in combinations of a 5'-3' sense strand primer with a 3'-5' anti-sense strand primer. Primer designation numbers ending in "3" (e.g., cox1B4483), represent sense strand primers for which nucleotide synthesis occurs off the 3' end of the primer. Primer designation numbers ending in "5" (e.g., cox1B5665), represent anti-sense strand primers for which nucleotide synthesis occurs off the 5' end of the primer. Thus, cox1B4483 and cox1B566 can be used together as primer pairs to amplify a *cox*1 gene intron. The same applies for the *cox*2 primers and for the *nad*1 primers. However, not all combinations of 3' and 5' primer pairs will necessarily work in PCR. In some cases, the distance between the 3' and 5' primers is too great for successful amplification.

### Example 3

### Use of Intronic region-specific Primer Pairs in PCR with Fungal DNA Templates.

Fungi representing 11 genera and 24 species were tested as DNA templates in a PCR using the four intron amplifying primer pairs derived from the *cox* 1 gene discussed in Example 2. These fungi are phylogenetically distinct and many are of agronomic significance. Fungi found in humans were included as convenient Ascomycete "outgroups."

Courtesy permits for transport of pathogen DNA were obtained from USDA-APHIS (Permit 34327) and from the California Department of Food and Agriculture (Permit # 1719). Results were obtained from the following isolates: 3 isolates of *Puccinia graminis*; 1 isolate of *P. coronata* and *P. horiana*; 1 isolate each *Tilletia indica*, *T. horrida*, *T. tritici*, and *T. species (spp.)*; 1 isolate of *Lycoperdon pyridome*; 1 isolate each of *Fusarium moniliforme* and *F. graminearum*, 3 isolates of *Aspergillus fumigatus* and 1 isolate each of *A. flavus*, *A. nidulans*, and *A. niger*; 2 isolates of *Cryptococcus neoformans;* 3 isolates each of *Saccharomyces cerevisiae*, *Candida albicans*, *C. glabrata*, *C. krusei*, *C. parapsilosis*, and *C. tropicalis.* The strains were recent field isolates obtained as DNA from Dr. Les Szabo, CDL, USDA-ARS, St. Paul, MN. Additional fungal samples were obtained from Dr. Mary Palm, USDA-APHIS, Mycology Laboratory, Beltsville, MD, Dr. Jon Duvick, Plant Pathologist, Pioneer Hi-Bred International, Johnston, IA, and Ms. Pat Nolan, Plant Pathologist, San Diego County Agriculture Commission. Fungal isolates from humans were obtained as DNA from Dr. Brad Cookson, U of WA, Seattle.

PCR reaction conditions for cox1B8103 + cox1B8975 primer pairs are as follows: Reaction mix contained 1 U AMPLITAQ^{®} polymerase (Perkin-Elmer), 50 mM KCI, 10 mM Tris-HCl (pH 8.3), 0.1 mM each dNTP (Ultrapure, Amersham-Pharmacia Biotech), 0.5 µM each primer, 50 to 100 ng DNA template. Reaction cocktail was heated to 80°C for 2 min in GENEAMP^{®} 9600 PCR machine (Perkin-Elmer), then 2.0 mM MgCI₂ was added for a total volume of 20 µL. PCR was performed for 35 cycles (94°C, 30 sec denature, 43°C, 30 sec anneal, 72°C, 2 min extension), followed by 6 min extension at 72°C. PCR products were resolved by loading 5.0 µL of the reaction onto a 1% agarose gel (Low EEO, Fisher Scientific) prepared in 1X TBE buffer and subjected to electrophoresis at 10 V cm⁻¹, then visualized by ethidium bromide staining.

PCR results using the cox1B8103 + cox1B8975 primer pair and the *cox*1b11703 + *cox*1B12965 primer pair are summarized in Table 1 and Table 2, respectively. Some of the products were cloned and sequenced to confirm their origin from the target exon as indicated.

Based on sequence motifs, all of the amplified introns are Group I introns and all except one contain at least one.ORF based on analysis using MacVector v.5.0.2 (Oxford Molecular Group, Oxford, UK). Both homologous and non-homologous introns are amplified using the cox1B8103 + *cox*1B8975 primer pair. Homologous introns from *T. indica*, *T. tritici*, and *L. pyriforme* are inserted at base 839 (on the *cox*1 consensus alignment), which is the known site of an intron in *Saccharomyces douglasii* (*cox*1 intron 2; GenBank accession # M97514) and *Podospora anserina* (*cox*1 intron 8; GenBank accession # X55026). Introns in *T. horrida* and *C*. *tropicalis* are inserted at base 850, and are homologous to introns from *S*. *cerevisiae* (*cox*1/*oxi*3 intron 4 GenBank accession # V00694), *P. anserina* (*cox*1 intron 9; GenBank accession # X55026), and *Pichia canadensis* (*cox*1 intron 2; GenBank accession # D31785).

In the tables below, *P. horiana* failed to yield a product with the primer pair *cox*1B8103 + *cox*1B8975 and *C*. *tropicalis* failed to yield a product with the primer pair *cox*1B11703 + *cox*1B12965, suggesting that the primers span an intron insertion site unique to *P. horiana* or *C*. *tropicalis,* respectively. Alternatively, an intron is present in each of these cases, but too large for resolution under the conditions used. Neither the single *P*. *graminis* or *F. moniliforme* isolate, nor the three isolates of *C*. *krusei, C. albicans, T. glabrata, A. fumigatus,* and *A. flavus,* or the two isolates of *C*. *neoformans* contain an intron in the *cox*1 gene in the region flanked by the *cox*1B8130 and *cox*1B8975 primers. The remainder of the isolates tested with these primers have an intron, and with the exception of *T. tritici,* of greater than 900 bp.

**Table 1. Results of PCR using cox1B8103 + cox1B8975**

| Species | Isolate | | | | Product^{a} | | Intron^{b} | Comments^{c} |
|---|---|---|---|---|---|---|---|---|
| | | | | | | | | |
| *P. graminis* | CRL78 | | ~90 bp | | | | | |
| *P. horiana* | 1 | | none | | | | | |
| *L. pyriforme* | ATCC46442 | | | 1547 bp | | 1459 bp blastp:nr 9e⁻¹⁹ *cox1* intron = | | |
| *T. indica* | | 1 | | 1523 bp | | 1435 bp blastn:nr 4e⁻⁴¹ *cox*1 *P. anserina* | | |
| *T. tritici* | | 1 | | 372 bp | | 291bp | | blastn:nr 3e⁻¹² *cox*1 *P.* |
| *anserina* | | | | | | | | |
| *T. horrida* | 1 | | 1060 bp | | 972 bp | | blastn:nr 1e⁻¹³⁸ *cox*1 *Peperomia* | |
| *S. cerevisiae* | AB 1380 | | | ~1000 bp | | ~920 bp expected size for *S*. *cerevisiae* | | |
| | | | | | | | *cox*1I4 intron | |
| *C. albicans* | 1 | | 88 bp | | none | | | |
| *C. albicans* | 2 | | 88 bp | | none | | | |
| *C. albicans* | 3 | | 88 bp | | none | | | |
| *C. glabrata* | 1 | | 88 bp | | none | | | |
| *C. glabrata* | 2 | | 88 bp | | none | | | |
| *C. glabrata* | 3 | | 88 bp | | none | | | |
| *C*. *krusei* | 1 | | 88 bp | | none | | aligns to *cox*1 exon | |
| *C*. *krusei* | 2 | | 88 bp | | none | | aligns to *cox*1 exon | |
| *C. krusei* | 3 | | 88 bp | | none | | aligns to *cox*1 exon . | |
| *C. tropicalis* | 1 | | 1055 bp | | 968 bp | | blastn:nr *6e*⁻⁰⁷ *cox*1 *Marchantia* | |
| *C. tropicalis* | 2 | | 1055 bp | | 968 bp | | | |
| *C. tropicalis* | 3 | | 1055 bp | | 968 bp | | | |
| *C. neoformans* | 1 | | 88 bp | | none | | | |
| *C. neoformans* | | 2 | | 88 bp | | none | | |
| *Fusarium moniliforme* | | 1 | | 88 bp | | none | | |
| *A. flavus* | | 1 | | 88 bp | | none | | |
| *A. flavus* | | 2 | | 88 bp | | none | | |
| *A. flavus* | | 3 | | 88 bp | | none | | |
| *A. fumigatus* | 1 | | 88 bp | | none | | | |
| *A. fumigatus* | 2 | | 88 bp | | none | | | |
| *A. fumigatus* | 3 | | 88 bp 88 | | none | | | |
| *A. niger* | | 1 | | 1481 bp | | 1393 bp blastn:nr 1e⁻¹²⁵ *cox*1 *P.* | | |
| *anserina* | | | | | | | | |

| | | | | | | | | |
|---|---|---|---|---|---|---|---|---|
| ^{a}Product of primer pair; if no intron then expect 88 bp exon fragment ^{b}Intron size confirmed by cloning and sequencing ^{c}Database queries using intron sequence | | | | | | | | |

**Table 2. Results of PCR using cox1B11703 + cox1B12965**

| Species | Isolate | | | Product^{a} | | Intron^{b} | Comments^{c} |
|---|---|---|---|---|---|---|---|
| *P. graminis* | CRL78~130 bp | | | | | | |
| *P. graminis* | CRL71~130 bp | | | | | | |
| *P. horiana* | 1 | ~350 bp | | ~220 | | | |
| *A. nidulans* | 1 | 127 bp | | none | | | |
| *A. niger* | | 1 | 127 bp | | none | | |
| *S. cerevisiae* | AB1380 | | ~1000 bp | | ~870 bp expected size for *S*. *cerevisiae* | | |
| *cox115* | | | | | | | |
| *L.pyriforme* | 1 | 127 bp | | none | | | |
| *C. tropicalis* | 1 | none | | | | | |
| *C. tropicalis* | 2 | none | | | | | |
| *C. tropicalis* | 3 | none | | | | | |
| *P.fumosoroseus* | | 1 | 127 bp | | none | | |

| | | | | | | | |
|---|---|---|---|---|---|---|---|
| ^{a}Product of primer pair; if no intron then expect 127 bp exon fragment ^{b}Intron size confirmed by cloning and sequencing ^{c}Database queries using intron sequences | | | | | | | |

Isolates of different species of the same genus appear to have introns of very different and easily distinguishable lengths as exemplified for *Tilletia* and *Candida* in Table 1. These "intronic region-specific" primers yielded products in the *Puccinia, Tilletia, Aspergillus* and *Candida* species tested, and the products displayed length polymorphisms between species. The existence of optional introns and sequence differences within introns provides an additional level of potential polymorphisms, which may be exploited further.

### Example 4

### Establishing Taxa-Specific Mitochondrial Intronic Profiles using Fungal Isolates

Cereal diseases are caused by a wide range of fungi that includes all the major fungal subclasses. Identification profiles are developed for 43 taxa representing all the major fungal causing cereal diseases. The taxa used in this example represent the many of the prominent cereal pathogens, including many prominent wheat pathogens.

Species level profiles are possible for some of the genera that are represented by more than one species, such as *Puccinia, Tilletia,* and *Fusarium.* For specificity and sensitivity of detection at the level of species, one is limited by the number of isolates that can reasonably be sampled, and on the validity of the current pathogen taxonomy. The difficulties encountered in such efforts may persist even though the genomic regions targeted and the technological approach used may be appropriate.

DNA is extracted using a modification of Berres et al., Mycologia, 87:821-840 (1995). All reactions are expected to yield a PCR product, even if no intron is found. Only when the intron is too large for PCR or when an accession has multiple introns in a given region will no product be observed with the "intronic-region amplifying" primers (Figure 2). This instance could result in a false-negative conclusion, so primer pairs that yield no product are omitted from the collection of primer pairs used to generate the identification profile.

PCR is performed and the products are cloned and sequenced (Example 8). The purpose of cloning and sequencing the products of the "first generation" primers is twofold. First, it confirms that the product is derived from the intended target region, and second, it provides sequence information on which to base "second generation" primers that encompass exon sequence variation in cereal pathogens. The sequence information includes the intron and exon-intron boundaries.

Second generation primers are developed that have increased specificity for the given taxa, and that yield small PCR products. The second-generation primers are designed for higher stringency PCR. The small products can out-compete larger, arbitrary PCR products that might arise from the host genome. Small products also are amenable to existing automated TAQMAN^{®} as well as non-PCR amplification technologies such as NASBA, LCR, SDA and TMA.

Some of the first generation primers that are highly specific and yield short products are used for intron profiling of the fungal isolates. Two pairs of primers are chosen that together classify the important species, and, where necessary, a number of other primers are in reserve to use in cases of ambiguity or unexpected results. In this process, primer pairs are identified that distinguish species of some of the genera as well.

The sequence information identifies those introns that encode open reading frames. Monoclonal antibodies are raised against the unique ORFs to detect the intronic polymorphisms in an immunological-based assay.

### Example 5

### Using Intronic region-specific Primer Pairs to Identify Organisms in Natural Samples

### A. Validation using plant specimens:

This example describes how to screen intronic region-specific primer pairs suitable for field sample use by using mock natural samples. Mixtures of extracted fungal DNA and wheat DNA is used as templates in PCR to establish optimum reaction conditions, selectivity, and sensitivity of the primer pairs (i.e., a "mock field" experiment) using intronic region-specific primer pairs for fungal organisms. In the experiment, purified fungal DNA is added to uninfected wheat DNA. DNA also is extracted from actual field specimens of plants suspected of containing fungi. Fungal DNA templates are extracted from infected plant material using the protocol described in Beres et al., *supra*, (1995).

### B. Validation using human specimens:

Mixtures of extracted fungal DNA and human DNA are used to establish optimum reaction conditions, selectivity, and sensitivity of intronic region-specific primer pairs in PCR. Also, in "mock clinical" specimens, extracted fungal DNA is added to uninfected patient serum, blood, or blood cultures. DNA also is extracted from actual clinical specimens known to contain fungi.

Fungal DNA templates are extracted from serum using proteinase K digestion in the presence of Tween 20 (Yamakami et al., J. Clin. Microbiol., 34:2464-24 (1996), and from whole blood using Zymolase with removal of most human DNA after red cell lysis and proteolytic digestion of white blood cells (Einsele et al., J. Clin. Microbiol., 35:1353-1360 (1997), and the addition of benzyl alcohol to remove sodium polyanetholesulfonate (SPS) (Fredricks et al., J. Clin. Microbiol., 36(10):2810-2816 (1998), an inhibitor of PCR: The efficiency can be increased by adding high-speed cellular disruption according to Muller et al., J. Clin. Microbiol., 36(6):1625-1629 (1998), after proteolytic digestion to remove excess sample protein.

Routine blood cultures obtained in the diagnostic laboratory which are positive for microbial growth, and confirmed to contain yeasts by Gram stain examination, are subjected to DNA extraction using the methods disclosed herein and tested in PCR with intronic region-specific primers.

### Example 6

### Epidemiological Assays for Puccinia graminis.

This example discloses application of the present methods to identification of the infectious agent in Rust disease of wheat. Rust diseases in wheat involve different parts of the plant and are caused by several members of the genus *Puccinia.* These species differ in life cycles and levels of genetic diversity. Presently, rust diseases are controlled via corresponding resistance genes bred into commercial wheat varieties. Because cereal rusts have the potential to cause such severe crop loss, they are the subject of annual surveys performed under the auspices of the USDA. The surveys monitor both the titer and distribution of rusts, with particular attention to *P*. *graminis,* the causal agent of wheat stem rust.

Wheat stem rust is the most aggressive and severe of the wheat rusts and was responsible for dramatic crop losses (up to 70 to 90%) during epidemic outbreaks in the early 1950s (Knott, In: The Wheat Rusts-Breeding for Resistance, Springer-Verlag, NY, pp 1-37 (1989)). The durability of the resistance to *P. graminis* in modem wheat varieties has been facilitated by the near-eradication of barberry (*Barberis vulgaris*), the sexual-stage host of *P*. *graminis,* which has slowed the development of new races of the pathogen. Race designations reflect the status of avirulence and virulence alleles. The intron-targeted strategy described herein is directed to "race"-specific profiles in cases where race designation is fully concordant with genetic clusters defined by molecular approaches.

Homologous introns are amplified and then digested with restriction enzymes to yield sufficient length and restriction enzyme polymorphisms. Also, fragments are resolved on single-stranded conformational polymorphism (SSCP) gels where fragments containing different sequences migrate to different places in the gel, and may be isolated and sequenced if further discrimination is needed. This technique is useful for revealing sequence polymorphisms in tRNA intergenic spacers in bacterial subspecies. PCR products that differed by only 2 out of 70 bases show different mobilities when resolved on a SSCP gel.

Three geographically distinct *P*. *graminis* f. sp. *tritici* populations are examined by PCR using primers validated as described above, and template extracted by procedures outlined above. First, members of an asexual clonal population found in the Midwestern U.S. are tested. Eleven races groups are identified in this population based on traditional avirulence/virulence testing with a standard wheat varietal panel, though only nine genetic clusters are confirmed by RAPD fingerprints. Thus, at least three isolates from each of these groups are used. About 25 isolates from a second population found in the Pacific Northwest study and representatives of a third population found in the Northeastern U.S. also are included for completeness.

### Example 7

### Epidemiological assays for A. fumigatus and A. flavus

This example discloses application of the present methods to identification of an infectious human pathogen. Invasive aspergillosis caused by *A. fumigatus* and to a lesser extent by *A. flavus,* is one of the deadliest of fungal infections. An improved diagnostic test to determine the genetic relatedness of clinical and environmental isolates early in the course of an apparent outbreak of invasive aspergillosis should help to identify a specific cause of the outbreak.

Intron specific primers are developed as described above to identify a sufficient combination of common and optional introns such that a profile is established to differentiate individual isolates. If there is insufficient presence or length variability within intronic regions of *Aspergillus,* sequence variability of homologous introns can be exploited to develop isolate-specific profiles. An initial approach to reveal sequence specific differences is to amplify homologous introns and then digest with restriction enzymes and resolve on single-stranded conformational polymorphism (SSCP) gels. Fragments containing different sequences migrate to different places in the gel and are isolated and sequenced.

Whole blood and serum specimens from human patients are examined for the presence of fungal elements by PCR using intronic region-specific primers and template extracted by procedures disclosed above. The specimens include those obtained for routine laboratory studies of immunocompromised patients who are subsequently diagnosed with invasive aspergillosis by tissue biopsy, or are colonized with *Aspergillus,* but show no evidence of invasive disease (which serves as controls in these experiments).

### Example 8

### Confirmed Sequences of Fungal cox1 Mitochondrial Genes

This example discloses six sequences of mitochondrial introns of yeast. Four of the five sequences have open reading frames that could code for a protein (i.e., an IREP), the amino acid sequences of which are disclosed further ahead.

### 1. Intronic Nucleotide Sequences

### A. Cox1 intron from Lycoperdon pyriforme

The sequence of an intron from the *cox*1 mitochondrial gene was obtained from the organism *Lycoperdon pyriforme* (Strain: ATCC 46442). The sequence is a consensus from 3 clones of a single isolate, each sequenced in both directions. The clones were obtained by cloning amplified DNA using cox1B8103 + cox1B8975 primer pairs. The full cloned sequence represents 1547 bp (SEQ ID NO: 29), with the intron at nucleotide position 31-1489 (SEQ ID NO: 30) and with exonic sequence upstream at positions 1-30 (SEQ ID NO: 31) and downstream at position 1490-1547 (SEQ ID NO: 32).

SEQ ID NO: 29 (1-1547)
GAGATCCTATTTTATATCAACACTTATTCT
TAACAAAAACATTGTACACTATTCCTCTAGTAGCTAAGAATTCGACAAGCTCCC
GCGAGCCTTTCCAATTTGGCACATTTTTGACACTTTACAGTAAACGTTTTCCTAA
CGCTAAGGCTCCTAGTCAATCCTTTTTAGATTGGCTAGTGGGATTTTCGGAAGG
AGACGGTAGCTTTATAATCAACAGTCGTGGAACAGCTATTTTCGTGATTACACA
AAGTACACTTGATCTACAAGTTCTTAAGTATATTCAACGAACTCTAGGTTTTGG
TCGTGTAATTAAACAAGGACAACGAACTAGTCGTTTTGTAGTTGAAGACAACG
CCAGTGTNTGCACTGCTAGTTGCTCTATTTAATGGAAATCTAATTTTCACAACT
AAACAATCTAGCTTTGCTTTATTTCTTGAAGCCTTTAACAAAAGATCATTGTCTT
TGGCTACTCAAGCAGTAGAACTTAAACCGTCACTGATTACTCCTACTAGACTAA
GCATACACGATTTTTGGTTAGCAGGTTTTACAGACGCTGAAGGTTGCTTCAATT
GCTCATTATTAGGTAACTCAAACGCGTATAGATTCCGATTTCTTCTAGCACAAA
AAGGAGAAGTTAATCTAACTGTACTGACACAGCTTACTAAACTTATTGGAGGT
GTTGTTCGTAATCACTCTAAACTGGGAGTATACGAATTAACTGTCAATGGTGCT
CGAAACGTGGAACGAGTATTCAAATATTTCGATACTCATCCGTTACAAACCAA
AAAAGCTAATTCGTACCAAATATGGCGAGAAGTTCATGCTTCTATCCTTAAAGG
AGAACATCTGTTACCAGAGTCTCGAGCAGCACTGAAAGTCAAAGCAGCTACTA
TTAATAACATGAATTAGTGTACAACCCAACGGGAATAAAGGAAGTGGTTCAAT
GTAATATCTCTTACCTACCAGGCTAACTAGATTAGAGACAAGTTGTGAAACTCT
AATAGGCAGGTGTCTATTTTAATTCTAAAGACCTGTTAGAGTGAATAATATTTA

TACCACTATTCTAGTCCATATTATACAGGTTGTGTAATCTTTAGAGAAAAACAG
CTTAGCCTTTGTTGCAGCAGAGCAGCTAATAATATGCTTACCCCGACAGGCGTA
AGGATGAACAATTGTTCATTGGCGATACAAGTGAAAACGGTCAACGTTTGCTC
GAACCAAGACCGTCGGTAGTTTAAACTATCGCTACAGACTGGGTCACTTGTGG
GTGCCTGAAAAGGTGCTTAATGTACAGTCGATTCCTTATATTACACAAGGCTAT
TGTGCTCTTTATGAGATTAGGTTTTTAGGTTCCAACAGCCAAAGCCAGCAGTAG
TTTAGGCACTTTCGCGAGCCTAAATCTACCTGGCCTACTGGGCTATTAAGCATC
CAGCCTACAATAGTACATGGGCCCTAGAGAGAGCTAATAAATCTAGGGTTTTA
GGGGATG
GGTTTTTTGGTCATCCAGAAGTTTATATTTTAATTATACCAGGATTTGGGATAGT
ATG

The insertion site of the intron (SEQ ID NO: 30) is homologous to that of *Saccharomyces douglasii* cox 1 intron 2 (GenBank accession # M97514) and *Podospora anserina* cox 1 intron 8 (GenBank accession # X55026).

### B. Cox1 intron from Tilletia indica

The sequence of an intron from the *cox*1 mitochondrial gene was obtained from the organism *Tilletia indica* (Strain: BPI 794197-1, natural isolate from wheat). The sequence is a consensus from 3 clones of a single isolate, each sequenced in both directions. The clones were obtained by cloning amplified DNA using cox1B8103 + cox1B8975 primer pairs. The full cloned sequence represents 1523 bp (SEQ ID NO: 33), with the intron at nucleotide position 31-1465 (SEQ ID NO: 34) and with exonic sequence upstream at positions 1-30 (SEQ ID NO: 35) and downstream at position 1466-1523 (SEQ ID NO: 36).

SEQ ID NO: 33 (1-1523)
GAGATCCTATTTTATATCAACACCTATTCT
CACTACTAAAAGTAGTTATTCTAATTCTATCTATTTACTTTTTCCAGGTTAAGCT
GAATGAGCCAACCACAAATACTTTTTCCTTTCATAATTTTACCCAACAATTTTC
ATCATTTTATCCTTCTAAACAAATACCTACTTTTTCTTTCCTAGAATGGCTTGTA
GGATTTACTGAAGGAGATGGCTGTTTTGTTATGAGCACTCGTGGTAACTGTATG
TTTGTTATTACACAATCTACTAAGGATATTCAAGTTCTTCATTTTATTCAAGATA
AACTAGGATTTGGTCGTGTTATTAAACAAGGACATTCTACATCTCGTTTTATTG
TTCAGGATAATAAGAATCTTTATCTACTTCTACATCTGTTTAATGGTAATCTAGT
ACTTCCTACTAAAATAGAAAGTTTTAAAAAGTTTATGGAGATATTTATCAAAAA
TTCATCTAATTATTCGATTACTCCAATTAGTGTTTGACGAACAACACCTAGTTGT
AATGACGCTTGAATTAGCGGATTTACAGATGCTGAAGGATGTTTTACTTGTTCT
CTACTTGGTAATTCTACAGCATATCGATTTCGTTTCATGCTTAGTCAAAAAAAT
GAGAAAAATAAGTGTGTACTAGATCATATTGCTTTTCTACTAAATGGAAAAGT
ACGACCTCACTCTATTCAAGGAGTGTATGAACTAACTGTAAACGGAATTTGTAA
TAATAAAGGAGTAGTACAATACTTTGATAAATATAAACTTTACACTAAAAAG

CAAGTTCATATCTACTATGGAAAGAAGTATCAGAGGATCTTAAAGATGGAAAA
CATCTTTCTGAAAGTACTCGTCTAATTATGAAAGAAAAGGTAATAAAAATCAA
TAGTTAGAAATAGTATATAATCTATCCCACGGAATAAAGGGTGTGGTTCTAC
ATAATTTTTATAGTTAATTTAAAATTTTATATTCCGACGCCTTCAGAGCGATTR
GAATAAATAAAACTAAATTGCCTCTGGGGTCAACGTGTAAAAACATAATAACT
ATAAAAAAAGAGCGAAATTTTATTAGGCAGGTGGTATTTTAATATAATGTAAA
GACCTAATATGATAAAGAGATATTCTCTACCACTACTCTAGTCCATGTCGTATA
AATCTGTGTAACCTTTAGAGGAAAACAGGTTTTAAGTATGTTTATGCCCACAGG
CATAAAGTGATTCTAAAAAATCATCGGCAATACAAGTGAAAACGGTCAACGTA
TATTCGTATGAAGACCGTCGGCAGTCTAAACTGTCGCTACAGACTGGGTCACTT
GTGGGTACCTGAAATGGTGCTTAATGTACAGTCGGCTTTCTCTAATGGTAAAAT
CATTACACAAGGTTATTCTCTCTATAAGAGGTCAGAATAGTACAGGGATTTCTA
AGAGAACTGATAAATTAGAAATTTGGGAAAGTG
GGTTCTTCGGTCATCCTGAAGTTTATATCCTGATTATACCAGGATTTGGGATAG
TAAG

The insertion site of the intron (SEQ ID NO: 33) is homologous to that of *Saccharomyces douglasii* cox 1 intron 2 (GenBank accession # M97514) and *Podospora anserina* cox 1 intron 8 (GenBank accession # X55026).

### C. Cox1 intron from Tilletia horrida

The sequence of an intron from the *cox*1 mitochondrial gene was obtained from the organism *Tilletia horida* (Strain: BPI 802756-1, natural isolate). The sequence is a consensus from 3 clones from a single isolate, each sequenced in both directions. The clones were obtained by cloning amplified DNA using cox1B8103 + cox1B8975 primer pairs. The full cloned sequence represents 1060 bp (SEQ ID NO: 37), with the intron at nucleotide position 42-1013 (SEQ ID NO: 38) and with exonic sequence upstream at positions 1-41 (SEQ ID NO: 39) and downstream at position 1014-1060 (SEQ ID NO: 40).

SEQ ID NO: 37 (1-1060)

GAGATCCTATTTTATATCAACATCTTTTTTGGTTCTTTGGT
CGAATATGGCCCGATATACCTATATTCAGAAGGGTATATATGAATTACACTGTA
TGCTGGAAATATCTGTTTAATGTTATTTCTACTATCATCATAAGAGGTATTATTA
CGAGCATATCCCGATATAGTAAAAATGAAATAACGAAGATACAATCAGCAGGT
AACCAACGACGCTCTATAAGCAGTCTAGTAGGAACCACAGAGACTATACGTGT
AACAACTTTTTCAACCACTTTTGGACAATGGCTAGCTGGCGTTATTGATGGCGA
TGGAAGTCTACAACTGAGTAAACAAGGCTATACAAGTCTTGAAATCACTATGG
GACTTGAAGATCTTCCTCTACTTCGTTATATTCAAGATAAACTTGGAGGATCTA
TTAAAATGCGAACGGAAGCCAAAGCTTATCGATATCGTCTACATAATAAAAGA
GGTATGATTACTATGATCAACTACATAAACGGAAATATTCGACATTCATCACGA
CTTACACAACTTCACCGAGTATGTTAACAACTTCATATACCTATCATGGAACCG

ATTCCACTAACGAATGATAATTACTGGTTTGCAGGATTTTTGATGCAGAAGGT
ACTATTACGTTTAGTTTCAAGAATGAATATCCTCAACTAAGCATACGAGTATCT
AATAAAAACATGGAAGACGTTCAGTGGTATAAAAATATATTTGGAGGCTATAT
CTATTTTGATAGTAGTCAATATGGTCATTATCAATGGTCAGTGCAAAGACGTAA
TGATGTTATAAGAATGAGAAGATATTTCAAGAATAAATGTAAAAGTCATAAAT
CAAACCGATTTTTCCTTATATCGGATTATTATCAACTTTCAGATCTAAAAGCAT
ATAAAAAAGAGAGTTAATATAATAATCTGTGGCACTATTTTGTCCAAAAGTGG
GACAAATTAAGTTGAAGATAAAGTCCATTTTATTTTACTGTGTAATATAGTAAA
AAAAAG
CATCCCGAAGTTTATATTCTAATTATACCAGGATTTGGGATAGTAAG

The insertion site of the intron (SEQ ID NO: 37) is homologous to that of *Saccharomyces cerevisiae cox*1/*oxi*3 intron 4 (GenBank accession #V00694), *Podospora anserina cox*1 intron 9 (GenBank accession #X55026) and *Pichia canadensis cox1* intron 2 (GenBank accession #D31785).

### D. Cox1 intron from Tilletia tritici

The sequence of an intron from the *cox*1 mitochondrial gene was obtained from the organism *Tilletia tritici* (Strain: T-1, natural isolate from wheat). The sequence is a consensus from 3 clones of a single isolate, each sequenced in both directions. The clones were obtained by cloning amplified DNA using cox1B8103 + cox1B8975 primer pairs. The full cloned sequence represents 372 bp (SEQ ID NO: 41), with the intron at nucleotide position 31-321 (SEQ ID NO: 42) and with exonic sequence upstream at positions 1-30 (SEQ ID NO: 43) and downstream at position 322-372 (SEQ ID NO: 44).

SEQ ID NO: 41 (1-372)
GAGATCCTATTTTATATCAACACCTGTTCT
CACTACTAAGACTAGTTATTCTAATTCTATCTATTTATTTTTCCAGCTTACGCA
GGATCAACAAACCATAAATACCTTTTCCTTTCATAATTTTACTGAACAATTTAA
AACCACATCATTTTTCCCTTCTAAACAAGTACCTACTTCTTCTTTTCTAGAATGG
TTTGTAGGATTTACTGAAGGAGACGGCAGTTTTGTTGTAAGCACTCGTGGTAAC
TGTATGTTTGTTATTACACAATCTACTAAGGATATTCAAGTTCTTCATTTTATCT
TTGCTTTACGGCTCCGCG
ANTTATATATAATAAAAAAGTTCAAGATAAACCAGGATTTGGGATAGTAAG

The insertion site of the intron (SEQ ID NO: 42) is homologous to that of *Saccharomyces douglasii* cox 1 intron 2 (GenBank accession # M97514) and *Podospora anserina* cox 1 intron 8 (GenBank accession # X55026).

### E. Cox1 intron from Candida tropicalis

The sequence of an intron from the *cox*1 mitochondrial gene was obtained from the organism *Candida tropicalis* (isolate from human). The sequence is a consensus from 2 clones each from a separate isolate, each sequenced in both directions. The clones were obtained by cloning amplified DNA using cox1B8103 + cox1B8975 primer pairs. The full cloned sequence represents 1055 bp (SEQ ID NO: 45), with the intron at nucleotide position 42-1009 (SEQ ID NO: 46) and with exonic sequence upstream at positions 1-41 (SEQ ID NO: 47) and downstream at position 1010-1055 (SEQ ID NO: 48). 
SEQ ID NO: 45 (1-1055)

GAGATCCTATTTTATATCAACACCTCTTCTGATTCTTCGGT
CAAGGTTGGCCCTTTGTAATACCCTTATTACATACGCATTACACTATATGCTGG
AAACTCCTATGTACATCGTACATAGCTTACTTAACTACTCTAGGTATCAGTCTA
CTCCTAGCCCCTAGAGTAAAAAGGTTAAGAGATAGTAGCAATACTAGCAGTGA
TGCAGCAGAKAACCAACGGTTCATATTCCAAGCTATTAATGCCTATGAACTCA
GTAGATATTTCAGAGACTACACGTGTAACTGTATCCCCTTCTACGGACCCATTC
CATCAATGATTAGCTGGTCTAATCGATGCTAATGGTGCCTTTAAAATCACTCAT
AAATCACAAGTAAATTGTGAGATAATAGTGCCTCAGAACGAGGAAAGAATGTT
AAGAGTTATTCAAGACAAGTATGGTGGTTCTATCAGGCTTAGATCAGGTGATC
GTACCCTTCGTTACAGATTACAAGATAAAGCTAGTGTAATCACCTTAATACAAC
ATGTTAATGGTAACCTTCATACTCCTTTAAGATTAAGCCAACTACATCGGGTAT
GTCCTCTACTTAATATAGAGGCTAACATGCCTATACCTTTAACCATATTTAATG
GTTGATTTATGGGCTATTTTGATGGTAAAGGTAACATCAGATGTAGAGTACCTA
ATATCTACTTAAGTGCTACAGGTAAAGCTGCAGTAAGTCTTCAAGGTTTTGTTG
ATGTTTTTGGTGGTGAGATAGTATACCGTAGAGCCAGCHATGGTTCATATACAT
GGAAACTATCCCGTCGACCTAGTGTGCTGTTATTTATGAGGTATCAGAMATGA
CATATATCACAGTCAACAMMGCAGCGGAGATTGGGCTTAATGAGAAAGTCTAT
CACTTAATTTACATGGAGAAAAGTGGGGATTTAAAARGATTTTCTCTGTTAAAG
ACATGAGTWTTATTCCATAATAAATGAAAATAAATGCAGAAGATATAGTCCAT
ACG
CATCCTGAGGKTTATATCCTGATTATACCAGGATTTGGGATAGTWAG
 The insertion site of the intron (SEQ ID NO: 46) is homologous to that of *Saccharomyces cerevisiae cox*1/*oxi*3 intron 4 (GenBank accession #V00694), *Podospora anserina cox*1 intron 9 (GenBank accession #X55026) and *Pichia canadensis cox*1 intron 2 (GenBank accession #D31785).

### F. Cox 1 intron from Aspergillus niger

The sequence of an intron from the *cox*1 mitochondrial gene was obtained from the *organism Aspergillus niger* (isolate from human). The sequence is from 2 clones of a single isolate, each sequenced in both directions. The clones were obtained by cloning amplified DNA using cox1B8103 + cox1B8975 primer pairs. The full cloned sequence represents 1481 bp (SEQ ID NO: 55), with the intron at nucleotide position 31-1423 (SEQ ID NO: 56) and with exonic sequence upstream at positions 1-30 (SEQ ID NO: 57) and downstream at position 1424-1481 (SEQ ID NO: 58).

SEQ ID NO: 55 (1-1481)
GAGATCCTATTTTATATCAACATCTTTTCT
CAAGAGATATTTAATTAATTGTTTAATATTAACAATTCTAGCTTCAATAGTAA
AGATTAATAAATCAAATTTAAGTTTTAAATTTAATTATAGTACTTTCATAAATA
AATTTRATTTTTCAAATTTTTATATAAAATTTTCTAATTATTTACCTAATAATAC
TTTACCTTCAGAAAAATTCTTGACTTGATTTATAGGATTCACAGAAGGTGAGGG
GTCATTTATAGTAAATAATAGAGGTGATCTTTGTTTTGTTATTACACAAAAAAC
TATAGATATTGAAATATTAGAATTTATAAAAGAAACTTTAGGTTTTGGTAAAGT
AATTCAACAATCTAAATTAACTAGTAGATATGTTACACAAAACAAAAAAGAAA
TAGAAATACTTATTCATTTGTTTAATGGTAATCTTATATTACCAAGTAGAAAGA
TAAAATTTGAAAATTTCATTAAAGGATTTAATATTTGAATAGGTAAAGGTAGA
ATAAAATTAGATCCTGTTGAATTAAAACATAATTTTATTTTACCTAGTTTAAAT
AATAGTTGATTGGCAGGTTTTACTGATGGGGAAGGCTGTYTTACTTGTTCTATA
GGTAAAGACAAAGGATTTAGTTTTAATTTTAATATTGCTCAAAAATGAGAGGA
AAATATTGAAGTATTACAACATCTTTGTACTTTATTTAATGGAGGAATAGTCTC
AAAACATAGTGTGGATAATGTAAATGAATTTAGAATAGGAGGATTAAAAAATT
GTAAAAATATATTTCCCTATTTTGATACTTATACATTATTAACTAAAAAATCTA
CTAGTTATATTTTATGAAAAGAAATATATGAAGATTTGTTAAAAAAATATCATT
TAGACCCAATTAAAAGGGTAGAGATGATTGAAAAAGCTAGATTGATAAATAAA
ATTAATTAATTAAAATATTAGGGAAAAAAAGTAAAGGTTTAACGTGCAAGTTT
TGAAGCTCTTAGGACAGATGTAAAAGGATATAAGATCCAAAAGAGCAAATATT
CTATAATGAATATACCTTATACTTAGTTAATGTTTAGTTATTACTACTTGCAACT
CTTAAGTGTAACGTATATATAATTTGGTATATATTGTTATACTTATCAATTAATA
TATAATTGATAAAAGGAAAAGTTAGTATAAACATTAGCGATACTAGTGTTAAC
GGTCAATAAATTTTCATGTTTAAAGACCGTCGGTTATTTAAGTGACCGCTACAG
ACTGGTTCACTGGTAGGTGGCTGAAATGCTGCTTAATGTACAGTCGGTTCCTTC
CATATTTTATATATGCACAAGCCCAGAATTATATAATTACTGGTACCTGGATTT
AATAAATGAACATCAATATATTGATGAGAAGTTAAATTTGAAGGAATG
GATTCTTCGGACATCCGGAAGTTTACATCTTAATTATACCAGGATTTGGGATAG
TAAG

The insertion site of the intron (SEQ ID NO: 56) is homologous to that of *Saccharomyces douglasii* cox 1 intron 2 (GenBank accession # M97514) and *Podospora anserina* cox I intron 8 (GenBank accession # X55026).

### 2. Intronic Open Reading Frame Sequences

MacVector v. 5.0.2 was used for open reading frame (ORF) analysis of the intronic sequences. Search options were set for all possible start/start codons using the yeast mitochondrial genetic code and a minimum of 100 amino acids. The amino acid sequence can vary depending upon the genetic code used for translation. In addition, the intronic sequences and adjacent upstream and downstream exons sequences were analyzed using the same search options to identify potential readthrough, or continuous ORFs. None were found. The intronic sequence ORFs are described below:

### A. Cox1 intron from Candida tropicalis

One ORF was identified and located from base 202 to 903 in the first frame of the plus strand shown as SEQ ID NO: 45, and is translated below using the yeast mitochondrial genetic code.

SEQ ID NO: 49 (CtropFrame1+/202-903 of SEQ ID NO: 45)
MQQXTNGSYSKTLMPMNSVDISETTRVTVSPSTDPFHQWLAGTIDANGAFKITHKS
QVNCEMMVPQNEERMLRVIQDKYGGSIRTRSGDRTTRYRLQDKASVITLMQHVN
GNTHTPLRLSQTHRVCPTTNMEANMPMPLTMFNGWFMGYFDGKGNIRCRVPNIY
LSATGKAAVSTQGFVDVFGGEMVYRRASXGSYTWKTSRRPSVTLFMRYQXWHM
SQSTXQRRLGLMRKSIT

### B. Cox1 intron from Tilletia horrida

Two ORFs were identified in the cloned intronic region shown as SEQ ID NO: 37 (i.e., the plus strand). ORF1 is located from base 81-548 in the third frame (SEQ ID NO: 50) while ORF2 is located from base 570-914 in the third frame (SEQ ID NO:51). Each of the ORFs are translated below using the yeast mitochondrial genetic code.

SEQ ID NO: 50 (ThFrame3+/81-548 of SEQ ID NO: 37)
MNYTVCWKYTFNVISTIIMRGIITSMSRYSKNEMTKMQSAGNQRRSMSSTVGTTET
MRVTTFSTTFGQWTAGVIDGDGSTQTSKQGYTSTEITMGTEDTPTTRYIQDKTGGS
IKMRTEAKAYRYRTHNKRGMITMINYMNGNIRHSSRTTQTHRVC

SEQ ID NO: 51 (ThFrame3+/570-914 of SEQ ID NO: 37)

MEPIPTTNDNYWFAGFFDAEGTITFSFKNEYPQTSMRVSNKNMEDVQWYKNMFG
GYIYFDSSQYGHYQWSVQRRNDVMRMRRYFKNKCKSHKSNRFFTMSDYYQTSD
TKAYKKES

### C. Cox1 intron from Lycoperdon pyriforme

One ORF was identified in the minus strand of the intronic region shown as SEQ ID NO: 29. For reference, SEQ ID NO: 52 is the complement of SEQ ID NO: 29 (i.e. the minus strand), shown in a 5'-3' direction and numbered from 1-1547 (i.e., a reverse complement sequence). The ORF (SEQ ID NO: 53) is located from base 646-1254 of SEQ ID NO: 52. The ORF is translated below using the yeast mitochondrial genetic code.

SEQ ID NO: 53 (LpyFrame1-/646-1254 of SEQ ID NO: 52)
MLLMVAALTFSAARDSGNRCSPLRMEAWTSRHIWYELAFLVCNGWVSKYLNTRS
TFRAPLTVNSYTPSLEWLRTTPPMSLVSCVSTVRLTSPFCARRNRNTYAFELPNNEQ
LKQPSASVKPANQKSCMTSTVGVISDGLSSTAWVAKDNDTLLKASRNKAKTDCLV
VKIRFPLNRATSSAXTGVVFNYKTTSSLSLFNYTTKT

### D. Cox1 intron from Tilletia indica

One ORF was identified, and located from base 225 to 899 in the third frame of the plus strand, shown as SEQ ID NO: 33, and is translated below using the yeast mitochondrial genetic code.

SEQ ID NO: 54 (TiFrame3+/225-899 of SEQ ID NO: 33)
MSTRGNCMFVITQSTKDIQVTHFIQDKTGFGRVIKQGHSTSRFIVQDNKNTYTTTHT
FNGNTVTPTKMESFKKFMEMFIKNSSNYSITPISVWRTTPSCNDAWISGFTDAEGCF
TCSTTGNSTAYRFRFMTSQKNEKNKCVTDHIAFTTNGKVRPHSIQGVYETTVNGIC
NNKGVVQYFDKYKTYTKKASSYTTWKEVSEDTKDGKHTSESTRTIMKEKVMKIN
S

### E. Cox1 intron from Tilletia tritici

No ORFs were identified in the *Tilletia tritici* intron sequence. Analysis of this intron was repeated using a minimum of 50 amino acid search option; no ORFs were identified.

### F. Cox 1 intron from Aspergillus niger

One ORF was identified, and located in from base 3 to 950 in the third frame of the plus strand, shown as SEQ ID NO: 55, and is translated below using the mold mitochondrial genetic code.

SEQ ID NO: 59 ( AnFrame3+/3-950 of SEQ ID NO: 55)
DPILYQHLFSRDILINCLILTILASIVKINKSNLSFKFNYSTFINKFXFSNFYIKFSNYLP
NNTLPSEKFLTWFIGFTEGEGSFIVNNRGDLCFVITQKTIDIEILEFIKETLGFGKVIQQ
SKLTSRYVTQNKKEIEILIHLFNGNLILPSRKIKFENFIKGFNIWIGKGRIKLDPVELK
HNFILPSLNNSWLAGFTDGEGCXTCSIGKDKGFSFNFNIAQKWEENIEVLQHLCTLF
NGGIVSKHSVDNVNEFRIGGLKNCKNIFPYFDTYTLLTKKSTSYILWKEIYEDLLKK
YHLDPIKRVEMIEKARLINKIN

### Example 9

### Screening Assays: Identification of target introns used in the design of in vivo assays for the screening of Compounds that modulate organelle intron-encoded maturase activity

This example shows the selection of a candidate intron containing open reading frames with putative maturase activity from our IREP database, designated Anil. The An intron 1 IREP, Anig 3/950, is a 315 aa IREP from the opportunistic human pathogen Aspergillus niger. Located in an intronic region of the cox1 gene, Anig 3/950 shares no amino acid sequence identity with other IREPs in our database. However, Anig 3/950 shares 25% amino acid sequence identity with a probable maturase from the corresponding intronic region in Schizosaccharomyces pombe and 22% amino acid identity with an intron in the corresponding intronic region in S. cerevisiae found in GenBank.

For puposes of illustration we will describe the screening assays using this target. In this example of an in vivo assay, two DNA constructs are introduced into yeast cells. One construct is designated the "maturase activity donor" or "mad" construct. "Mad" contains the Ancox1i1maturaseUni cassette, comprised of a portion of the preceding Ancox1e1 exon sequence and the cox1i1 ORF sequence that have each been converted from their original organellar genetic code (Org) to the universal genetic code (Uni), downstream of an inducible (or constitutive, but preferably inducible) promoter. The construct also contains selectable antibiotic or nutritional genes, for example. Various alternative constructs are apparent to those of skill in the art to ensure a suitable level of expression depending on the desired effect.

For one of skill in the art, codon conversion is accomplished by well established strategies such as site-directed mutagenesis, and the like. Accordingly, conversion of the genetic codes is accomplished by synthesis of oligonucleotides derived from each strand from positions spaced along the sequence. PCR is used to correct the changes to the new code and the PCR products are ligated together to form the contiguous sequence, Ancox1i1maturaseUni in this example. The conversion to the universal code allows for expression in the cytoplasm or nucleus. Several options are available to one of skill in the art to confirm proper translation and expression of the synthethic maturase. Accordingly, antibodies raised against the maturase (as described in detail in the preceding diagnostic methods section) are used to detect expression of Ancox1i1maturaseUni by hybridization and Western blotting.

The second construct is designated the "maturase activity target" construct, or "mat". "Mat" contains the Ancox1i1ORFUni cassette, comprised of constitutive (or inducible) promoter, a portion of Ancox1e1 exon sequence and the Ancox1i1ORF sequence, fused to a reporter gene such as the green fluorescent protein gene (gfp). In this case only the Ancox1e1 sequence is converted from Org to Uni. A further, ancoxliORF may be altered, for example, engineered by inserting stop codons, to abolish its potential maturase activity. The construct also contains selectable antibiotic or nutritional genes, different from those used in the Mad construct. Conversion of the preceding exon sequence provides for proper readthrough to the reporter gene. In this example, gfp is expressed only when maturase activity supplied in trans from the Mad construct facilitates splicing of Ancox1i1ORF from the Mat construct, thereby permitting expression of gfp.

Mad and Mat constructs are introduced into yeast cells via standard transformation methods known to one of skill in the art, for example, via transformation using lithium acetate (Ausubel et al 1997:Short Protocols in Molecular Biology). Transformants are identified by growth on selective medium.

As a control, the activity of the synthetic maturase and its target are monitored by assays of the Mat construct reporter gene as shown in Table 1.

Candidate compounds are identified from, for example, a small molecule combinatorial library by including the compound in the growth medium. Without regard to the specific nature of any particular compound, outcomes anticipated in this assay as shown in Table 1: yeast fail to grow; yeast grow and do not express gfp; yeast grow and do express gfp. Compounds that yield the first outcome are lethal to yeast and are less desirable as therapeutic candidates because their mode of action may disrupt a target that is common in the target organism and its host. Compounds that yield the second outcome disrupt maturase activity and are candidates for therapeutic uses because growth of the organism is specifically inhibited. Finally, compounds that yield the third outcome fail to disrupt maturase activity but do not inhibit the organism's growth and so are not candidates for therapeutic uses.

As another control, the transcription and translation of unrelated genes will be monitored by, for example, Northern hybridization and Western hybridization, respectively. This control confirms a compound's modulation of maturase activity.

In the assay described in this example the compound is required to enter a fungal cell, in this case yeast. However, other screening assays utilizing different constructs and host cells can be easily envisioned by one of skill in the art, for example, a three-hybrid assay system [RNA 6:1882-1894 (2000)]

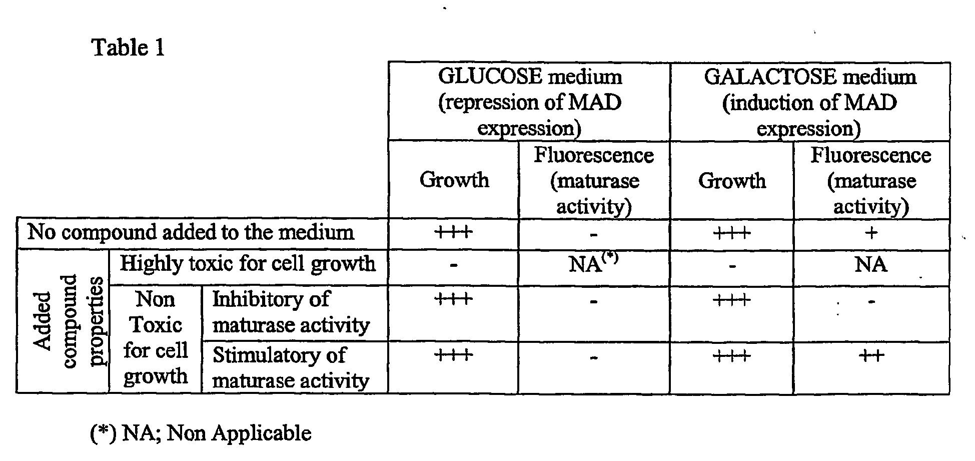

### Example 10

### Identification of target introns

This example shows the selection of candidate introns containing open reading frames with suitable maturase activity used for identifying therapeutic agents. IREPs are available from our database of intronic region amplification products derived from several genes for many assessions. These products are cloned, sequenced, and the sequences analyzed using Omiga 2.0 (Oxford Molecular Group) to identify the open reading frames. Some ORFs are continuous with the reading frame of the preceding exon, whereas others are fully contained within the intron. Next, DNA sequences of the intronic region are translated using the appropriate genetic code. Codes for mitochondrial genomes in yeast and molds are available from GenBank (http://www.ncbi.nml.nih.gov). A default minimum length of 50 amino acids is used in ORF analysis. GenBank is searched for homologies to the amino acid sequence using Blastn and Blastx (Altschul et al. 1990). TBlastx analysis is also done against the mitochondrial database. From these analyses we find the predicted homologies to exons from which the PCR primers had been derived. In some of the IREPs, we observe amino acid similarities with known intron-encoded proteins.

Two such candidate introns encoding open reading frames from our database are given as examples. A candidate IREP from Aspergillus niger (designated Anig 3/950) is 316 aa and is located in an intronic region of the cox1 gene. It shares no amino acid sequence identity with other IREPs in our database. However, Anig 3/950 shares 25% amino acid sequence identity with a probable maturase from the corresponding intronic region in Schizosaccharomyces pombe and 22% amino acid identity with an intron in the corresponding intronic region in S. cerevisiae found in GenBank.

A Candida tropicalis IREP (234 aa and designated Ctp202/903) is in the same intronic region of cox1 as Anig3/950. Ctp202/903 and Anig3/950 share no amino acid identity. However, Ctp202/903 shares 35% amino acid identity with a S. cerevisiae maturase and a Pichia canadensis hypothetical protein from the corresponding intronic region.

Additional candidate IREPs in our database are derived from the same intronic region of cox1, but not necessarily from a homologous insertion site, as well as from different intronic regions in cox1 and in other mitochondrial genes. In this manner confirmed IREPs with maturase activity from other species of Aspergillus, Candida, Paecilomyces, Histoplasma, Coccidioides, Cryptococcus, and other clinically significant fungi, are identified as potential targets for modulation by therapeutic agents. IREPs with maturase activity from species of Tilletia, Puccinia, Fusarium, Phytophthora, and other agronomically significant fungi, are identified as potential targets for modulation by fungicidal agents.

The examples set forth above are provided to give those of ordinary skill in the art with a complete disclosure and description of how to make and use the preferred embodiments of the compositions, and are not intended to limit the scope of what the inventors regard as their invention. Modifications of the above-described modes for carrying out the invention that are obvious to persons of skill in the art are intended to be within the scope of the following claims.

## Claims

1. A method for screening an agent for modulating cellular activity of a non-human organism, wherein said organism contains an intron comprising a nucleic acid encoding a protein that effects intronic region encoded protein (IREP)-mediated post-transcriptional processing of RNA, said method comprising the steps of:
a) providing the protein in an assay for studying the effects of the protein on post-transcriptional processing of pre-mRNA; and
b) assaying for said effects in the presence of the agent.

2. A method according to claim 1, wherein said intron is a Group I or Group II intron.

3. A method according to claim 1 or 2, wherein said IREP is a maturase.

4. A method according to any one of the claims 1 to 3, wherein said organism is fungus, bacterium, plant or protozoan.

5. A method according to any one of the claims 1 to 4, wherein said agent inhibits growth of the organism.

## Patentansprüche

1. Verfahren zum Screening eines Wirkstoffes für die Modulation der zellulären Aktivität eines nicht-humanen Organismus, wobei der Organismus ein Intron enthält, welches eine Nukleinsäure umfaßt, die für ein Protein kodiert, das eine *intronic region encoded protein* (*IREP*)-vermittelte post-transkriptionelle Prozessierung von RNA bewirkt, wobei das Verfahren die Schritte umfaßt:
a) Bereitstellung des Proteins in einem Assay zur Untersuchung der Wirkungen des Proteins auf post-transkriptionelle Prozessierung von prä-mRNA; und
b) Untersuchung der Wirkungen in Gegenwart des Wirkstoffes.

2. Verfahren gemäß Anspruch 1, wobei das Intron ein Gruppe I- oder Gruppe II-Intron ist.

3. Verfahren gemäß Anspruch 1 oder 2, wobei das IREP eine Maturase ist.

4. Verfahren gemäß irgendeinem der Ansprüche 1 bis 3, wobei der Organismus ein Pilz, ein Bakterium, eine Pflanze oder ein Protozoe ist.

5. Verfahren gemäß irgendeinem der Ansprüche 1 bis 4, wobei der Wirkstoff das Wachstum des Organismus inhibiert.

## Revendications

1. Procédé de criblage destiné à identifier un agent qui module l'activité cellulaire d'un organisme non humain, dans lequel ledit organisme contient un intron comprenant un acide nucléique codant pour une protéine qui influence le remaniement post-transcriptionnel de l'ARN médié par la protéine codée par ladite région intronique, ledit procédé comprenant les étapes suivantes :
a) utiliser la protéine dans un dosage pour étudier les effets de la protéine sur le remaniement post-transcriptionnel du pré-ARNm ; et
b) doser lesdits effets en présence de l'agent.

2. Procédé selon la revendication 1, dans lequel ledit intron est un intron du Groupe I ou du Groupe II.

3. Procédé selon la revendication 1 ou 2, dans lequel ladite IREP est une maturase.

4. Procédé selon l'une quelconque des revendications 1 à 3, dans lequel ledit organisme est un champignon, une bactérie, une plante ou un protozoaire.

5. Procédé selon l'une quelconque des revendications 1 à 4, dans lequel ledit agent inhibe la croissance de l'organisme.
